# EUROPEAN PATENT SPECIFICATION

(11) **EP 1 093 379 B1**
(45) Date of publication and mention of the grant of the patent: **03.01.2007**
(21) Application number: 99932296.9
(22) Date of filing: 06.07.1999
(51) Int. Cl.: A61K 31/40, A61K 38/00, A61K 38/08, A61P 29/00

(54) **USE OF INHIBITORS OF PROTEIN KINASE C EPSILON TO TREAT PAIN**
VERWENDUNG DER PROTEIN-KINASE-C-EPSILON-INHIBITOREN ZUR BEHANDLUNG VON SCHMERZEN
UTILISATION D'INHIBITEURS DE LA PROTEINE KINASE C EPSILON DANS LE TRAITEMENT DE LA DOULEUR

(30) Priority: 06.07.1998 US 91755 P; 09.10.1998 US 103763 P
(43) Date of publication of application: 25.04.2001
(73) Proprietor: The Regents of the University of California, Oakland, CA 94607-5200 (US)
(72) Inventor: Messing, Robert O., Foster City, CA 94404 (US); Levine, Jon D., San Francisco, CA 94118 (US)
(74) Representative: Kremer, Simon Mark
(86) International application number: PCT/US1999/015265
(87) International publication number: WO 2000/001415

(56) References cited:
- WO-A-97/15575
- US-A- 5 783 405
- CESARE P ET AL: "Specific involvement of PKC-epsilon in sensitization of the neuronal response to painful heat." NEURON, JUL 1999, 23 (3) P617-24, XP000869683 UNITED STATES
- KHASAR, S. G. ET AL: "A novel nociceptor signalling pathway revealed in Protein Kinase C epsilon mutant mice" NEURON, vol. 24, no. 1, September 1999 (1999-09), pages 253-260, XP000869769
- KHASAR, S.G. ET AL: "Epinephrine produces a beta-adrenergic receptor mediated mechanical hyperalgesia and in vitro sensitization of rat nociceptors" JOURNAL OF NEUROPHYSIOLOGY, vol. 81, no. 3, March 1999 (1999-03), pages 1104-11112, XP000869664
- HUNDLE, B. ET AL: "An inhibitory fragment derived from Protein Kinase C epsilon prevents enhancement of Nerve Growth Factor responses by ethanol and phorbol esters" THE JOURNAL OF BIOLOGICAL CHEMISTRY, vol. 272, no. 23, 1997, pages 15028-15035, XP002119149 cited in the application
- HUNDLE, B. ET AL: "Overexpression of epsilon-Protein kinase C enhances Nerve Growth Factor-induced phosphorylation of mitogen-activated protein kinases and neurite outgrowth" THE JOURNAL OF BIOOGICAL CHEMISTRY, vol. 270, no. 50, 1995, pages 30134-30140, XP000867400
- LEWIN, G.R. ET AL: "Nerve growth factor and nociception" TRENDS IN NEUROSCIENCE, vol. 16, no. 9, 1993, pages 353-359, XP000870184
- LEWIN, G.R. ET AL: "Nerve growth factor-induced hyperalgesia in the neonatal and adult rat" THE JOURNAL OF NEUROSCIENCE, vol. 13, no. 5, 1993, pages 2136-2148, XP000870190
- JOHNSON, J. A. ET AL: "A Protein Kinase C translocation inhibitor as an isozyme-selective antagonist of cardiac function" THE JOURNAL OF BIOLOGICAL CHEMISTRY, vol. 271, no. 40, 1996, pages 24962-66, XP000867211 cited in the application
- DATABASE CHEMABS [Online] CHEMICAL ABSTRACTS SERVICE, COLUMBUS, OHIO, US AN: 126:5425, HATTORI, KIYOSHI: "Role of spinal N-type Ca channels and protein kinase C activities in modulating hyperalgesia. Determination of spinal amino acid release and pain related response" XP002129369 & KYUSHU SHIKA GAKKAI ZASSHI , vol. 50, no. 5, 1996, pages 789-806,
- DATABASE DISSERTATION ABSTRACTS [Online] UMI AN: 01237083, MAO, JIANREN: "Excitatory central mechanisms of post-injury neuropathic pain" XP002129370 & DISSERTATION ABSTRACTS INTERNATIONAL, vol. 53, no. 04-B, 1992, page 1726
- SLUKA K.A. ET AL: "Capsaicin-induced sensitization of primate spinothalamic tract cells is prevented by a protein kinase C inhibitor" BRAIN RESEARCH, vol. 772, no. 1-2, 1997, pages 82-86, XP000869915
- AHLGREN, S.C. ET AL: "Protein Kinase C inhibitors decrease hyperalgesia and C-fiber hyperexcitability in the Streptozotocin-diabetic rat" THE JOURNAL OF NEUROPHYSIOLOGY, vol. 72, no. 2, 1994, pages 684-692, XP000869507 cited in the application
- SLUKA KA ET AL: "The effects of G-protein and protein kinase inhibitors on the behavioral responses of rats to intradermal injection of capsaicin." PAIN, vol. 71, no. 2, 1997, pages 165-178, XP000869590
- CODERRE TJ ET AL: "Intracellular messengers contributing to persistent nociception and hyperalgesia induced by L-glutamate and substance P in the rat formalin pain model." EUROPEAN JOURNAL OF NEUROSCIENCE, AUG 1 1994, 6 (8) P1328-34, vol. 6, no. 8, 1994, pages 1328-34, XP000869767
- MUNRO F.E. ET AL: "Evidence for a role of protein kinase C in the sustained activation of rat dorsal horn neurons evoked by cutaneous mustard oil application" NEUROSCIENCE LETTERS, vol. 170, no. 2, 1994, pages 199-202, XP000869681
- OHSAWA, MASAHIRO ET AL: "Modulation of the formalin-induced nociceptive response by diabetes: possible involvement of protein kinase C" BRAIN RESEARCH, vol. 803, no. 1,2, 1998, pages 198-203, XP000869976
- CODERRE TJ: "Contribution of protein kinase C to central sensitization and persistent pain following tissue injury." NEUROSCIENCE LETTERS, vol. 140, no. 2, 1992, pages 181-184, XP000869761
- YASHPAL K ET AL: "Noxious thermal and chemical stimulation induce increases in 3H-phorbol 12,13-dibutyrate binding in spinal cord dorsal horn as well as persistent pain and hyperalgesia, which is reduced by inhibition of protein kinase C." THE JOURNAL OF NEUROSCIENCE, vol. 15, no. 5 part 1, 1995, pages 3263-3272, XP000869657
- LIN Q ET AL: "Inhibition of primate spinothalamic tract neurons by spinal glycine and GABA is reduced during central sensitization." THE JOURNAL OF NEUROPHYSIOLOGY, AUG 1996, 76 (2) P1005-14, vol. 76, no. 2, 1996, pages 1005-1014, XP000869667
- MCGUIRK, S.M. ET AL: "G-protein mediation in nociceptive signal transduction: an investigation into the excitatory action of bradykinin in a subpopulation of cultured rat sensory neurons" NEUROSCIENCE, vol. 49, no. 1, 1992, pages 117-128, XP000869517 cited in the application
- MAO J. ET AL: "Increases in protein kinase C gamma immunoreactivity in the spinal cord dorsal horn of rats with painful mononeuropathy" NEUROSCIENCE LETTERS, vol. 198, no. 2, 1995, pages 75-78, XP000869679

## Description

### INTRODUCTION

### Background

For those who experience it, as well as for their families, communities and employers, pain is more than a minor inconvenience - it is a serious physical, emotional, social and economic burden. Because more than two million people in the United States alone are incapacitated by chronic pain on any given day (T. M. Jessell & D. D. Kelly, *Pain and Analgesia* in PRINCIPLES OF NEURAL SCIENCE, 3^{rd} edition (E. R. Kandel, J. H. Schwartz, T. M. Jessell, ed., 1991)), the number of people and entities bearing these burdens is quite large.

Unfortunately, current treatments for pain are only partially effective, and many also cause debilitating or dangerous side effects. For example, non-steroidal anti-inflammatory drugs ("NSAIDs") such as aspirin, ibuprofen and indomethacin are moderately effective against inflammatory pain but they are also renal toxins, and high doses tend to cause gastrointestinal irritation, ulceration, bleeding and confusion. Patients treated with opioids frequently experience confusion, and long-term opioid use is associated with tolerance and dependence. Local anesthetics such as lidocaine and mixelitine simultaneously inhibit pain and cause loss of normal sensation. Thus, there is a need for safe and effective treatments for pain.

Enhanced understanding of the molecular basis of pain should aid the development of pain medications. Pain is a particularly challenging subject because it is a perception based on signals received from the environment and transmitted and interpreted by the nervous system. Noxious stimuli such as heat and touch cause specialized sensory receptors in the skin to send signals to the central nervous system ("CNS"). This process is called nociception, and the peripheral sensory neurons that mediate it are nociceptors. Depending on the strength of the signal from the nociceptor(s) and the abstraction and elaboration of that signal by the CNS, a person may or may not experience a noxious stimulus as painful.

While pain is not enjoyable, it is crucial - without it, we would be oblivious to many environmental dangers. When our perception of pain is properly calibrated to the intensity of the stimulus, pain serves its intended protective function. However, certain types of tissue damage cause a phenomenon, known as hyperalgesia or pronociception, in which relatively innocuous stimuli are perceived as intensely painful because the person's pain thresholds have been lowered. Both inflammation and nerve damage can induce hyperalgesia. Thus, persons afflicted with inflammatory conditions, such as sunburn, osteoarthritis, colitis, carditis, dermatitis, myositis, neuritis, collagen vascular diseases (which include rheumatoid arthritis and lupus) and the like, often experience enhanced sensations of pain. Similarly, trauma, surgery, amputation, abscess, causalgia, collagen vascular diseases, demyelinating diseases, trigeminal neuralgia, cancer, chronic alcoholism, stroke, thalamic pain syndrome, diabetes, herpes infections, acquired immune deficiency syndrome ("AIDS"), toxins and chemotherapy cause nerve injuries that result in excessive pain. Apparently, the reduced pain thresholds characteristic of hyperalgesia are due to alterations in the way that nociceptors adjacent to the inflammation or damaged nerves respond to noxious stimuli. If the mechanisms by which nociceptors transduce external signals under normal and hyperalgesic conditions were better understood, it might be possible to identify processes unique to hyperalgesia that, when interrupted, could inhibit the lowering of the pain threshold and thereby lessen the amount of pain experienced. Since such a treatment for chronic pain would act at the level of the sensory afferent neurons, it would bypass the problems associated with drugs that act on the CNS. If the treatment incapacitated a transduction pathway specific to nociceptors and/or not involved in mediating other signals, then the potential for treatment-induced side effects would be small. The present specification discloses such a method of alleviating pain.

### SUMMARY OF THE INVENTION

The present specification discloses a method of lessening pain comprising administering to a subject in need thereof, an effective amount of an inhibitor of the ε isozyme of protein kinase C ("PKCε"). Also disclosed is the use of a PKCε inhibitor to decrease hyperalgesia, preferably without impairing nociception. The inhibitors are selective for PKCε and local administration of the inhibitor is preferred. One aspect of the invention is a method of measuring the ability of compounds to modulate pain as set out in the claims comprising selecting a test compound that modulates the activity of PKCε and administering said test compound in an animal model subject to determine whether pain is modulated. A further aspect of the invention is a pharmaceutical composition as set out in the claims comprising an inhibitor of PKCε and an analgesic agent that is not an inhibitor of PKCε. Additional aspects of the invention include the use of a modulator of PKCε, which is an inhibitor of PKCε, in the preparation of a medicament as set out in the claims. Such a medicament can be used for the treatment of pain. This invention is well suited to the treatment of subjects having acute or chronic pain caused by neuropathic or inflammatory conditions.

### BRIEF DESCRIPTION OF THE DRAWINGS

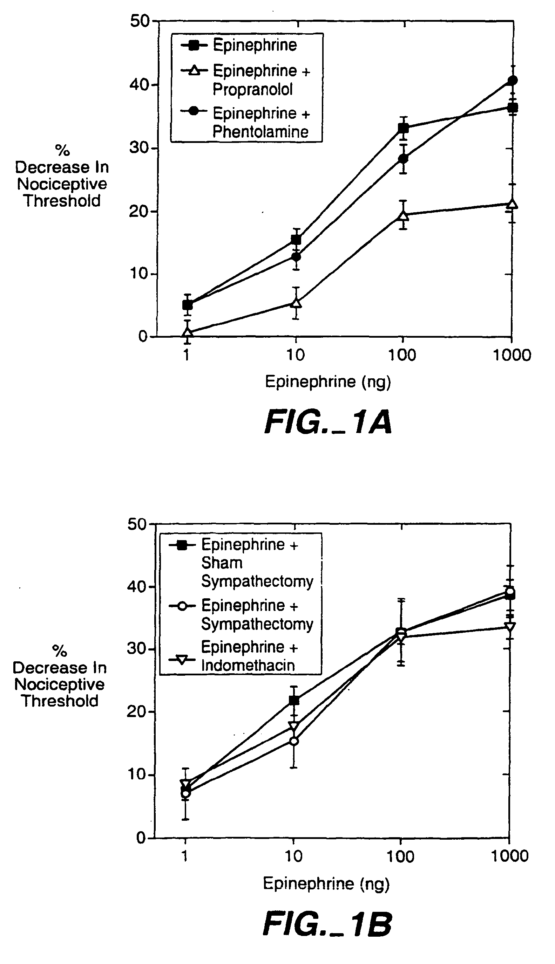

**Figure 1.** Effect of α- and β- adrenergic antagonists, sympathectomy and prostaglandin synthesis inhibitors on epinephrine-induced dose-dependent hyperalgesia in response to mechanical stimuli. Percent decrease in nociceptive thresholds due to injection of 1, 10, 100 and 1000ng of epinephrine following (**A**) no treatment (squares), propranolol injection (triangles), phentolamine injection (filled circles) and (**B**) sham sympathectomy (squares), sympathectomy (circles) and indomethacin injection (inverted triangles).

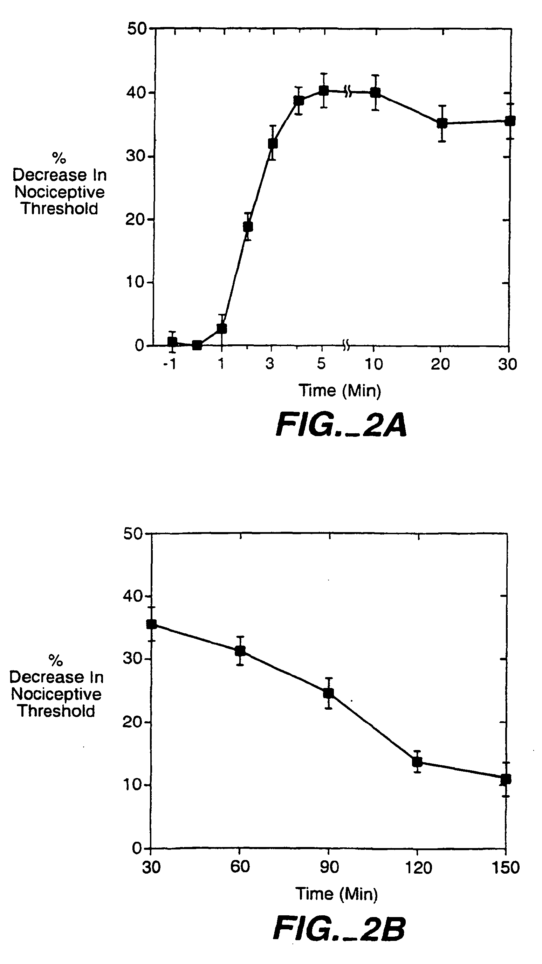

**Figure 2.** Latency and duration of epinephrine-induced hyperalgesia in response to mechanical stimuli. Percent decrease in nociceptive thresholds at indicated number of minutes following 1µg epinephrine injection.

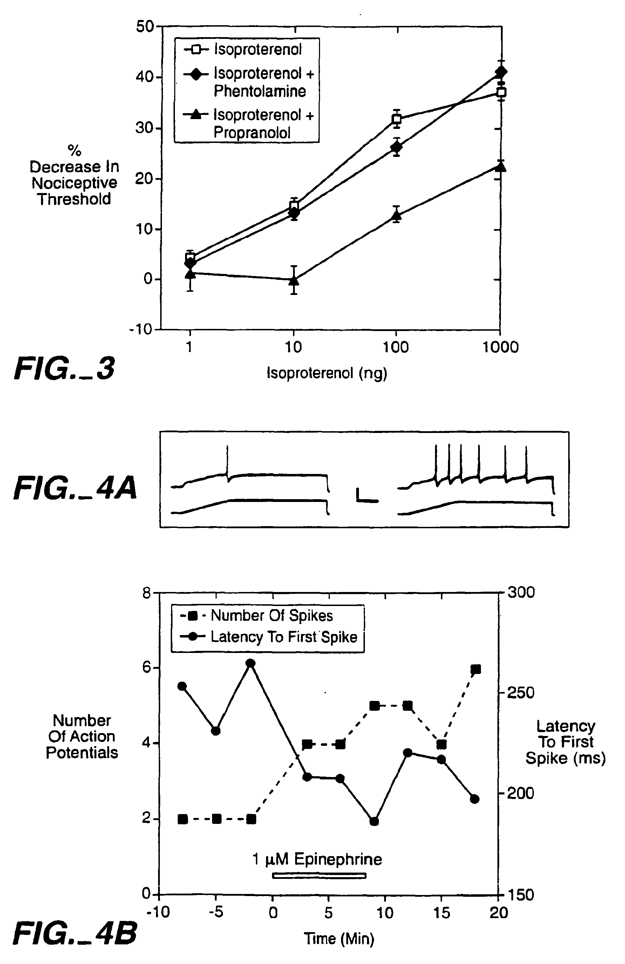

**Figure 3.** Effect of α- and β- adrenergic antagonists on isoproterenol-induced hyperalgesia in response to mechanical stimuli. Percent decrease in nociceptive thresholds due to injection of 1, 10, 100 and 1000ng of isoproterenol following no treatment (squares), phentolamine injection (diamonds) and propranolol injection (triangles).
**Figure 4** (**A**) Voltage traces from a cultured rat small diameter DRG neuron before and during exposure to 1µM epinephrine. (**B**) Effect of 1µM epinephrine over time on number of action potentials (squares) and latency to first action potential (circles) in another cultured rat small diameter DRG neuron.

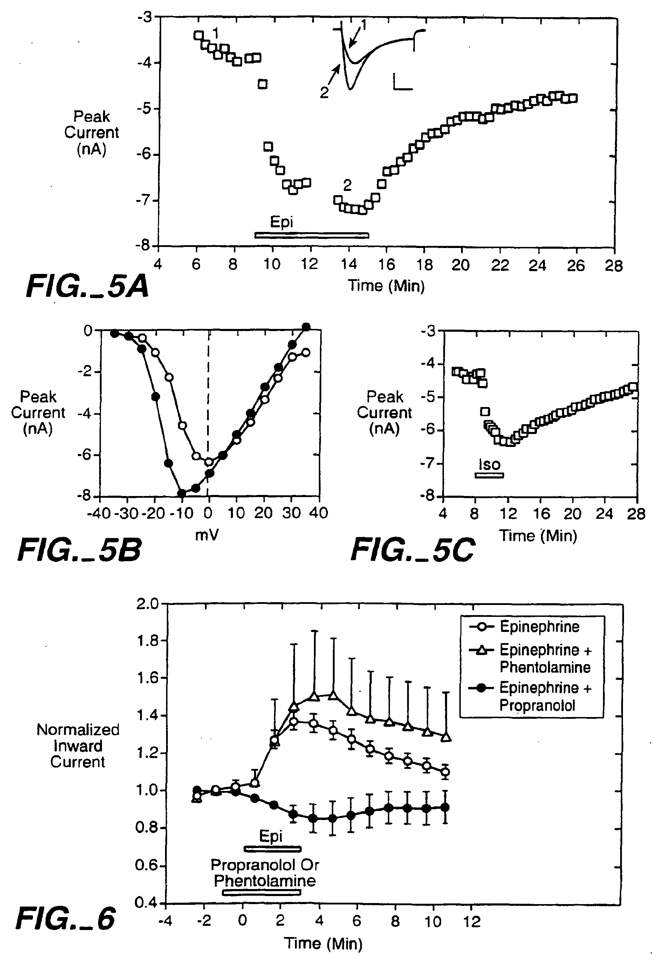

**Figure 5.** Effect over time of (**A**) 1µM epinephrine or (C) isoproterenol on peak tetrodotoxin-resistant sodium current ("TTX-RI_{Na} current"). (**B**) TTX-RI_{Na} current-voltage plots for no epinephrine (empty circles) and 1µM epinephrine (filled circles).
**Figure 6.** Effect of α- and β- adrenergic antagonists on epinephrine-induced potentiation of TTX-RI_{Na} current. Normalized inward current over time with perfusion of 1µM epinephrine alone (empty circles), 1µM epinephrine and phentolamine (triangles), and 1µM epinephrine and propranolol (filled circles).

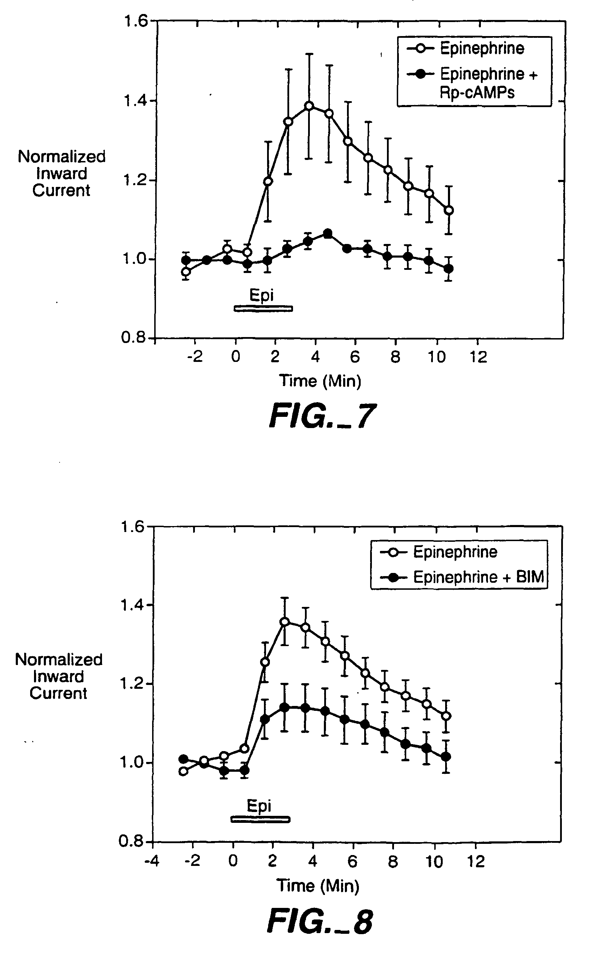

**Figure 7.** Effect of PKA inhibitor on epinephrine-induced potentiation of TTX-RI_{Na} current. Normalized inward current over time with perfusion of 1µM epinephrine alone (empty circles) or perfusion of 1µM epinephrine with Rp-cAMPs present in recording pipette (filled circles).
**Figure 8.** Effect of PKC inhibitor on epinephrine-induced potentiation of TTX-RI_{Na} current. Normalized inward current over time with perfusion of 1µM epinephrine alone (empty circles) or perfusion of 1µM epinephrine with BIM pretreatment (filled circles).

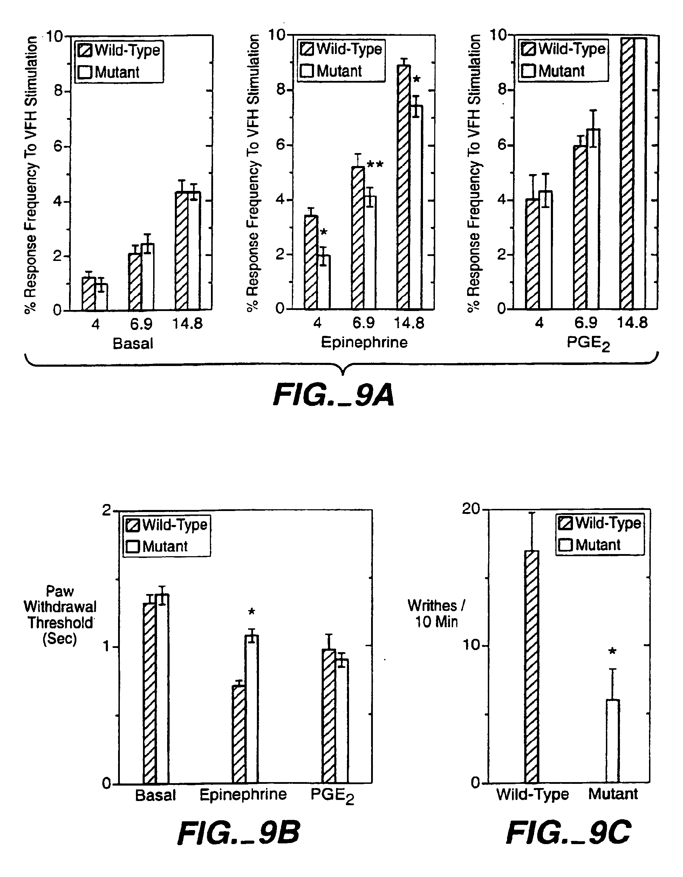

**Figure 9.** Mechanical, thermal, and chemical nociception and hyperalgesia in wild-type and PKCε-mutant mice. (**A**) The responses of wild-type (diagonal striped bars) and PKCε-mutant (white bars) mice to mechanical stimuli were examined by measuring paw withdrawal frequencies after von Frey hair ("VFH") stimulation of 4, 6.9 or 14.8gm intensity. VFH stimulation was preceded by no treatment ("basal"), epinephrine injection, or PGE₂ injection. (**B**) The tolerance of wild-type (diagonal striped bars) and PKCε-mutant (white bars) mice to thermal stimuli was determined by measuring length of exposure (in seconds) to a 50 watt radiant heat stimulus prior to paw withdrawal. Heat stimulation was preceded by no treatment ("basal"), epinephrine injection, or PGE₂ injection. (**C**) The ability of intraperitoneal acetic acid injection to induce abdominal contractions ("writhes") was examined in wild-type and PKCε-mutant ("knock-out") mice.

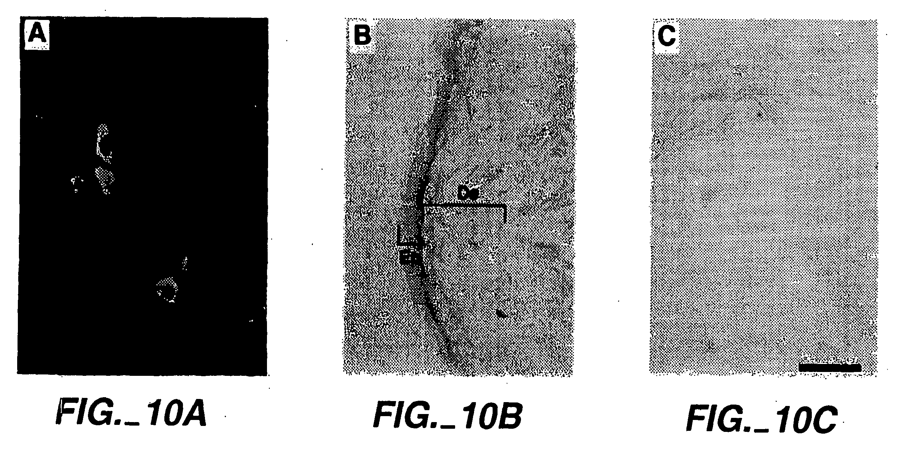

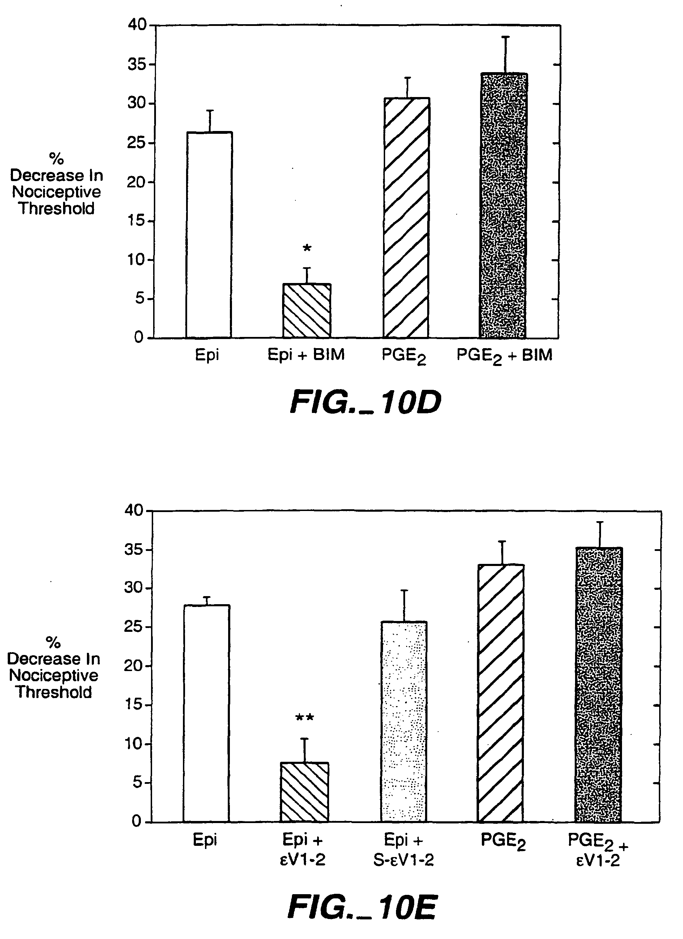

**Figure 10.** PKCε expression in rat DRG neurons and skin; inhibition in rats of epinephrine-induced hyperalgesia by a non-selective PKCε inhibitor and a selective PKCε inhibitor. Rat DRG neurons (**A**) and skin (**B** and **C**) were stained with PKCε antibody (**A** and **B**) and antibody preabsorbed with antigen (**C**). Bar = 50 µm (**A, B** and **C**); Ep = epidermis; De = dermis. Rats received intradermal injections of 100ng epinephrine ("epi") or 100ng PGE₂ in the wake of injections into the dorsal aspect of the hindpaw of (**D**) 1µg bisindolymaleimide I ("BIM"), or (**E**) sterile water followed either by 1µg εV1-2 peptide ("VIε1-2,") or 1 µg of a scrambled peptide ("S-VIε1-2"). The change in nociceptive threshold was tested by measuring the response to Ugo Basile stimulation.

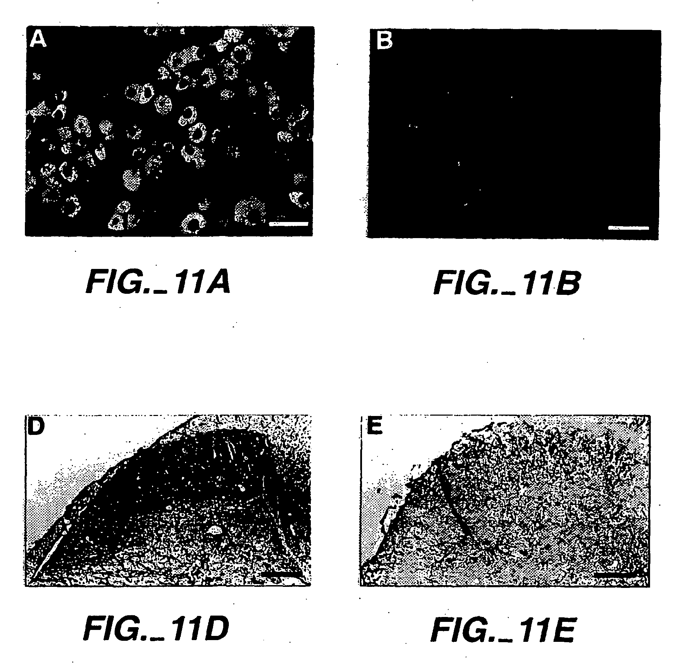

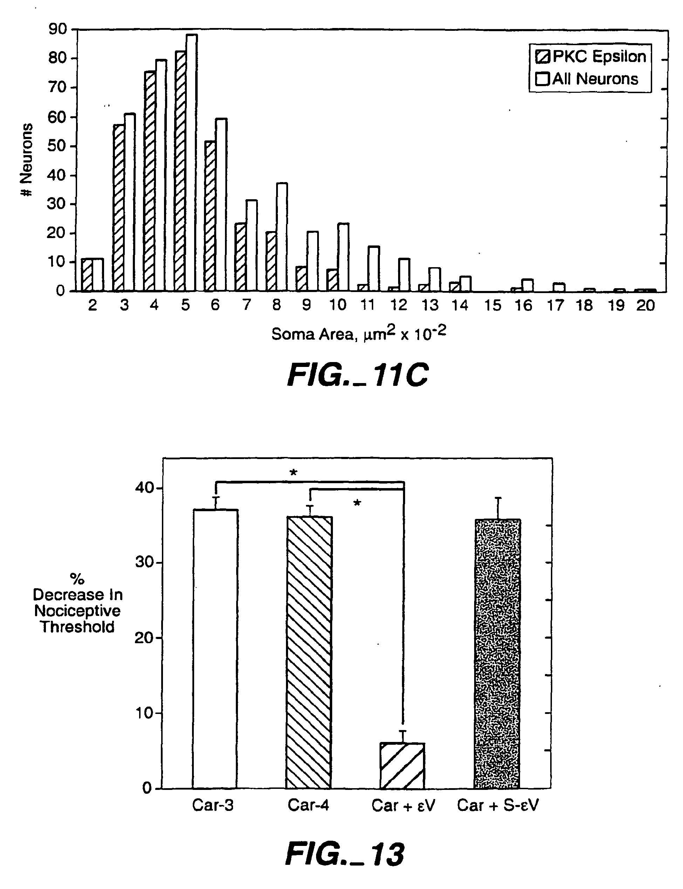

**Figure 11.** PKCε expression in mouse dorsal root ganglion ("DRG") neurons (**A** and **B**) and spinal cord (**D** and **E**). Wild-type (**A** and **D**) and PKCε-mutant (**B** and **E**) mouse tissues were incubated with anti-PKCe antibody. Bar = 50 µm (**A** and **B**) or 100 µm (**D** and **E**). (**C**) Size frequency histogram of PKCe-expressing and all DRG neurons.

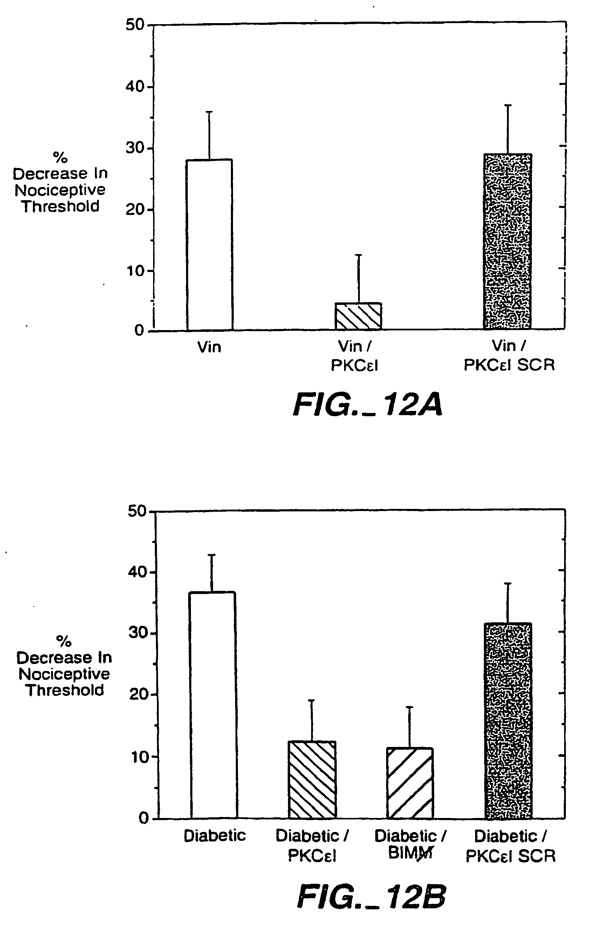

**Figure 12.** Effect of PKCε inhibitors on vincristine-induced hyperalgesia (A) or diabetes-induced hyperalgesia (B) in response to mechanical stimuli. Percent decrease in nociceptive thresholds following no treatment (white bars), injection of PKCε inhibitory peptide (diagonal striped bars), injection of a scrambled version of the inhibitory peptide (stippled bars) or injection of bisindolymaleimide I (reverse diagonal striped bar).
**Figure 13.** Effect of PKCε inhibitor on carrageenan-induced hyperalgesia in response to mechanical stimuli. Percent decrease in nociceptive thresholds following exposure to carrageenan alone (white and diagonal striped diagonal strip bars - hyperalgesia tested at 3 and 4 hours post carrageenan, respectively), exposure to carrageenan and injection of PKCε inhibitory peptide (reverse diagonal striped bar), or exposure to carrageenan and injection of a scrambled version of the inhibitory peptide (stippled bar). * indicates significantly different, p<0.0001.

### DESCRIPTION OF SPECIFIC EMBODIMENTS

Protein kinase C ("PKC") is a multigene family of phospholipid-dependent, serine-threonine kinases central to many signal transduction pathways. Molecular cloning studies have identified ten members of the PKC family. These family members, called isozymes, are encoded by nine different genes. The ten isozymes are designated as the α, βI, βII, γ, δ, ε, ζ, η, ι/λ. and θ isozymes (Y. Nishizuka, *Science* **258***,* 607-614 (1992); L. A. Selbie, C. Schmitz-Peiffer, Y. Sheng, T. J. Biden, *J Biol*. *Chem.* **268**, 24296-24302 (1993)). Based on sequence homology and biochemical properties, the PKC gene family has been divided into three groups: (i) the "conventional" PKCs, the α, βI, βII and γ isozymes, are regulated by calcium, diacylglycerol and phorbol esters; (ii) the "novel" PKCs, the δ, ε, θ and η isozymes, are calcium-independent, but diacylglycerol- and phorbol ester-sensitive; and (iii) the "atypical" PKCs, the ζ and í/λisozymes, are insensitive to calcium, diacylglycerol and phorbol 12-myristate 13-acetate. In addition, two related phospholipid-dependent kinases, PKCµ and protein kinase D, share sequence homology in their regulatory domains to novel PKCs and may constitute a new subgroup (F.-J. Johannes, J. Prestle, S. Eis, P. Oberhagemann, K. Pfizenmaier, *Biol. Chem.* **269**, 6140-6148 (1994); A. M. Valverde, J. Sinnett-Smith, J. Van Lint, E. Rozengurt, *Proc. **Natl.** Acad*. *Sci*. *USA* **91**, 8572-8576 (1994)).

It is well established that PKC family proteins play central roles in cell growth and differentiation. PKCs mediate the effects of peptide hormones, growth factors, neurotransmitters and tumor promoters by acting as secondary (downstream, intracellular) messengers for these signaling molecules (Y. Nishizuka, *Science* **233,** 305-312 (1986); Y. Takai, K. Kaibuchi, T. Tsuda, M. Hoshijima, *J*. *Cell*. *Biochem*. **29**, 143-155 (1985)). The identities of the PKC isozymes that transduce particular signals in specific cell types are still being determined. The α, βI, βII, γ, δ, ε and ζ isozymes have been implicated in the differentiation of nonneural cells (E. Berra, et al., *Cell* **74**, 555-563 (1993); J. Goodnight, H. Mischak, J. F. Mushinski, *Adv. Cancer Res.* **64**, 159-209 (1994); J. R. Gruber, S. Ohno, R. M. Niles, *J*. *Biol*. *Chem.* **267**,13356-13360 (1992); D. E. Macfarlane, L. Manzel, *J*. *Biol*. *Chem*. **269**, 4327-4331 (1994); C. T. Powell et al., *Proc. Natl. Acad. Sci. USA* 89, 147-151 (1992)). Recent studies, showing that the ε isozyme of PKC ("PKCε") is activated by nerve growth factor ("NGF") and mediates NGF-induced neurite outgrowth, were interpreted as indicating a role for PKCε in neuronal differentiation (B. Hundle, et al., *J*. *Biol. Chem.* **272**, 15028-15035 (1997)).

One of the key discoveries underlying the present invention is the demonstration that a specific isozyme of PKC, PKCε, acts in primary afferent nociceptors to mediate certain types of hyperalgesia. In particular, mice that lack functional PKCε protein ("PKCε-mutant mice") show reduced responses to acetic acid injections and decreased epinephrine-induced hyperalgesia in response to mechanical and thermal stimuli. Administration of a peptide that selectively inhibits PKCε also diminishes epinephrine-induced hyperalgesia, carrageenan-induced hyperalgesia and hyperalgesia associated with chemotherapy and diabetes, in response to mechanical stimuli. Because the magnitude of epinephrine-induced hyperalgesia was similar in rats treated with a non-selective PKCε inhibitor and those treated with the selective inhibitor of PKCε, PKCε appears to be the only PKC isozyme mediating epinephrine-induced hyperalgesia. Similarly, because the magnitude of diabetes-associated hyperalgesia was slightly less in rats treated with a non-selective PKCε inhibitor than in those treated with the selective inhibitor of PKCε, PKCε appears to be the major, but perhaps not the only, PKC isozyme mediating diabetes-associated hyperalgesia.

The discovery that PKCε mediates certain types of hyperalgesia was unexpected. Although there was evidence that the PKC family of proteins contributes to diabetic neuropathic hyperalgesia (S. C. Ahlgren, J. D. Levine, *J. Neurophys.* **72**, 684-692 (1994)) and to bradykinin-induced activation and sensitization of nociceptors (S. M. McGuirk, A. C. Dolphin, *Neuroscience* **49**, 117-28 (1992); L. M. Boland, A. C. Allen, R. Dingledine, *J. Neurosci.* **11**, 1140-9 (1991)), the roles of individual PKC isozymes in these processes were unexplored and unpredictable.

A particularly useful aspect of the present invention is the fact that inhibitors of PKCε can reduce hyperalgesia without affecting nociception or compromising other sensory perception. Thus, a person receiving a PKCε inhibitor should have relief from excessive pain stemming from innocuous stimuli while still being able to sense pain in response to intense stimuli. This aspect of the invention is based on experiments using mechanical and thermal stimuli to show that PKCε mediates epinephrine-induced hyperalgesia and carrageenan-induced hyperalgesia but is not involved in nociception (i.e., the basal response). The reduced writhing seen in PKCε-mutant mice injected with acetic acid in comparison to wild-type mice is consistent with PKCε having a role in inflammatory hyperalgesia but not nociception. The theory that acetic acid injection causes inflammation that results in hyperalgesia is supported by data showing that anti-inflammatory drugs (NSAIDs in these cases) inhibit the amount of writhing induced by acetic acid exposure (C. J. Niemegger, J. A. Van Bruggen and P. A. Janssen, *Arzneimittelforschung* **25**, 1505-1509 (1975); R. Vinegar, J. F. Truax and J. L. Selph, *Eur*. *J*. *Pharmacol*. **37**, 23-30 (1976); R. Bjorkman, *Acta Anaesthesiol*. *Scand*. *Supp*. **103**, 1-44 (1995)). The fact that administration of a selective inhibitor of PKCε nearly eliminates hyperalgesia otherwise caused by carrageenan, a well established inflammatory agent, further solidifies the role of PKCε in mediating inflammatory hyperalgesia.

As described above, the present specification describes the administration of an inhibitor of PKCε to lessen pain, prevent future pain, and/or inhibit heightened sensitivity to a noxious stimulus. Although any molecule that inhibits the PKCe isozyme is sufficient to lessen pain, molecules that selectively inhibit the PKCε isozyme are preferred because, as shown by PKCε-mutant mice, elimination of PKCε does not cause major developmental abnormalities or serious side effects. Since molecules also capable of inhibiting PKC isozymes other than PKCε interfere with the various functions performed by those isozymes, such nonselective inhibitors of PKCε, although they diminish pain, are likely to have many unwanted side effects.

There are many known inhibitors of PKCε that can be used in the instant invention. For instance, U.S. Patent No. 5,783,405 describes a large number of peptides that inhibit PKC isozymes. Of these, the εV1-1, εV1-2, εV1-3, εV1-4, εV1-5 and εV1-6 peptides are selective for PKCε and are preferred peptide inhibitors. Peptide εV1-2 is a particularly preferred inhibitory peptide. Small molecule inhibitors of PKC are described in U.S. Patent Nos. 5,141,957, 5,204,370, 5,216,014, 5,270,310, 5,292,737, 5,344,841, 5,360,818, and 5,432,198. These molecules belong to the following classes: N,N'-Bis-(sulfonamido)-2-amino-4-iminonaphthalen-1-ones; N,N'-Bis-(amido)-2-amino-4-iminonaphthalen-1-ones; vicinal-substituted carbocyclics; 1,3-dioxane derivatives; 1,4-Bis-(amino-hydroxyalkylamino)-anthraquinones; furo-coumarinsulfonamides; Bis-(hydroxyalkylamino)-anthraquinones; and N-aminoalkyl amides. Due to their relative ease of administration (for instance, transdermal delivery or ingestion are often feasible for small molecules but not peptides), small molecule inhibitors of PKCε are preferred over peptide inhibitors.

Additional inhibitors of PKCε can be identified using assays that measure the activation, intracellular translocation, binding to intracellular receptors (e.g. RACKs) or catalytic activity of PKCε. Traditionally, the kinase activity of PKC family members has been assayed using at least partially purified PKC in a reconstituted phospholipid environment with radioactive ATP as the phosphate donor and a histone protein or a short peptide as the substrate (T. Kitano, M. Go, U. Kikkawa, Y. Nishizuka, *Meth. Enzymol.* **124**, 349-352 (1986); R. O. Messing, P. J. Peterson, C. J. Henrich, *J. Biol. Chem.* **266**, 23428-23432 (1991)). Recent improvements include a rapid, highly sensitive chemiluminescent assay that measures protein kinase activity at physiological concentrations and can be automated and/or used in high-throughput screening (C. Lehel, S. Daniel-Issakani, M. Brasseur, B. Strulovici, *Anal*. *Biochem*. **244**, 340-346 (1997)) and an assay using PKC in isolated membranes and a selective peptide substrate that is derived from the MARCKS protein (B. R. Chakravarthy, A Bussey, J. F. Whitfield, M. Sikorska, R. E. Williams, J. P. Durkin, *Anal. Biochem.* **196**, 144-150 (1991)). Inhibitors that affect the intracellular translocation of PKCε can be identified by assays in which the intracellular localization of PKCε is determined by fractionation (R. O. Messing, P. J. Peterson, C. J. Henrich, *J*. *Biol*. *Chem.* **266**, 23428-23432 (1991)) or immunohistochemistry (U.S. Patent No. 5,783,405; U.S. Patent Application No. 08/686,796). To identify an inhibitor of PKCε, the assays should be performed with PKCε. The selectivity of such PKCε inhibitors can be determined by comparing the effect of the inhibitor on PKCε with its effect on other PKC isozymes.

Because PKCε is an intracellular protein, preferred embodiments of the invention involve pharmaceutically acceptable inhibitor formulations capable of permeating the plasma membrane. Small, apolar molecules are often membrane permeable. The membrane permeability of other molecules can be enhanced by a variety of methods known to those of skill in the art, including dissolving them in hypotonic solutions, coupling them to transport proteins, and packaging them in micelles.

PKCε inhibitors can be administered hourly, several times per day, daily or as often as the person experiencing the pain or that person's physician sees fit. Preferably, the administration interval will be in the range of 8 to 24 hours. The severity of the patient's pain can be taken into account when determining appropriate intervals for PKCε inhibitor treatments. PKCε inhibitor treatments can continue over the course of several days, one month, several months, one year, several years or the duration of the patient's lifetime. Alternatively, PKCε inhibitors can be administered on a one-time only basis provided that the pain is expected to be transient and it does not reappear. PKCε inhibitors should be administered at levels sufficient to reduce pain in the body of the patient. The skilled artisan will appreciate that increasing doses of PKCε inhibitors should be administered until the patient experiences pain reduction, and larger doses fail to effect greater pain amelioration. Administration could be set up such that the patient can control future dosages of PKCε inhibitor according to the amount of pain they experience.

Inhibitor dosage will vary according to many parameters, including the nature of the inhibitor and the mode of administration. For the εPKC-v1 peptide, a 150µg/ml intracellular concentration inhibited PKCε translocation and downstream effects of PKCε activation (U.S. Patent No. 5,783,405). Daily dosages in the range of 1µg/kg-100mg/kg of body weight, preferably 1µg/kg-1mg/kg and most preferably 10µg/kg-1mg/kg are contemplated for PKC inhibitors that are NN'-Bis-(sulfonamido)-2-amino-4-iminonaphthalen-1-ones or N,N'-Bis-(amido)-2-amino-4-iminonaphthalen-1-ones. Daily dosages in the range of 1µg/kg-100mg/kg of body weight, preferably 1µg/kg-40mg/kg and most preferably 10µg/kg-20mg/kg are contemplated for PKC inhibitors that are vicinal-substituted carbocyclics. Daily dosages in the range of 5-400mg/kg of body weight, preferably 10-200mg/kg and most preferably 10-50mg/kg are contemplated for PKC inhibitors that are 1,4-Bis-(amino-hydroxyalkylamino)-anthraquinones, Bis-(hydroxyalkylamino)-anthraquinones, or N-aminoalkyl amides. Daily dosages in the range of 0.1-40mg/kg of body weight, preferably 1-20mg/kg, are contemplated for PKC inhibitors that are 1,3-dioxane derivatives. Daily dosages in the range of 1-100mg/kg of body weight are contemplated for PKC inhibitors that are furo-coumarinsulfonamides.

PKCε inhibitors can be locally administered near the site of inflammation or peripheral nerve damage. Such local administration can be topical or by intradermal or subcutaneous injection. Systemic administration of a PKCε inhibitor represents another embodiment of the invention. Oral and intravenous injection are preferred types of systemic administration. Since PKCε appears to modulate pain at the level of the peripheral nociceptors and may perform other functions in the brain, local administration of PKCε inhibitors is more preferable than systemic administration because local administration should effectively treat pain in the vicinity with minimal deleterious effects on PKCε activity in distant locations. The methods described in the specification are useful for treating mammals in general and humans in particular.

A preferred embodiment of the present invention may involve the administration of a pharmaceutically acceptable formulation of an inhibitor of PKCε. A "pharmaceutically acceptable formulation" comprises one that is suitable for administering the PKCε inhibitor in a manner that gives the desired results and does not also produce adverse side effects sufficient to convince a physician that the potential harm to a patient is greater than the potential benefit to that patient. The basic ingredient for an injectable formulation is a water vehicle. The water used will be of a purity meeting USP standards for sterile water for injection. Aqueous vehicles that are useful include sodium chloride (NaCl) solution, Ringer's solution, NaCl/dextrose solution, and the like. Water-miscible vehicles are also useful to effect full solubility of the PKCε inhibitor. Antimicrobial agents, buffers and antioxidants are useful, depending on the need.

In preparing PKCε inhibitor compositions for this invention, one can follow the standard recommendations of well known pharmaceutical sources such as REMINGTON: THE SCIENCE AND PRACTICE OF PHARMACY, 19^{th} ed., (Mack Publishing, 1995). In general, the pharmaceutical composition of this invention is powder- or aqueous-based with added excipients that aid in the solubility of the PKCε inhibitor, the isotonicity of the composition, the chemical stability and the deterrence of microorganism growth. For oral administration, it is preferable to include substances that protect the PKCε inhibitor from degradation by digestive agents.

The invention may be used in the treatment of a patient having inflammatory pain. Such inflammatory pain may be acute or chronic and can be due to any number of conditions characterized by inflammation including, without limitation, sunburn, rheumatoid arthritis, osteoarthritis, colitis, carditis, dermatitis, myositis, neuritis and collagen vascular diseases. This use of the invention is based on experiments showing that administration of a PKCε inhibitor significantly diminishes both acute and chronic hyperalgesia resulting from exposure to the inflammatory agent carrageenan. The fact that PKCε inhibitors effectively reduce chronic hyperalgesia when administered long after the inflammatory agent (carrageenan in these experiments) indicates that persons who have long suffered pain associated with inflammatory diseases such as those listed above should experience at least partial pain relief following administration of an inhibitor of PKCε. In addition, administration of an PKCε inhibitor to a subject immediately prior to, during or after an inflammatory event can ameliorate both the acute pain and the chronic hyperalgesia that the subject would otherwise experience.

The invention may also be used in the treatment of a patient having neuropathic pain. Such patients can have a neuropathy classified as a radiculopathy, mononeuropathy, mononeuropathy multiplex, polyneuropathy or plexopathy. Diseases in these classes can be caused by a variety of nerve-damaging conditions or procedures, including, without limitation, trauma, stroke, demyelinating diseases, abscess, surgery, amputation, inflammatory diseases of the nerves, causalgia, diabetes, collagen vascular diseases, trigeminal neuralgia, rheumatoid arthritis, toxins, cancer (which can cause direct or remote (e.g. paraneoplastic) nerve damage), chronic alcoholism, herpes infection, AIDS, and chemotherapy. Nerve damage causing hyperalgesia can be in peripheral or CNS nerves. This use of the invention is based on experiments showing that administration of a PKCε inhibitor significantly diminishes hyperalgesia due to diabetes, chemotherapy or traumatic nerve injury.

It is believed that inhibitors of FKCε lesson pain by interfering with PKCε function in sensory afferent neurons (also known as nociceptors or primary afferent neurons). These neurons, a subset of small- and medium-diameter dorsal root ganglion neurons ("DRG neurons"), extend from the dermis, where their peripheral terminals are located, to the superficial laminae of the dorsal horn, where they synapse with CNS neurons. In sensory afferent neurons, PKCε is believed to be a secondary messenger transducing a response initiated by a noxious stimulus or a hyperalgesiainducing agent.

Some of the observations underlying the present invention were made in experiments in which hyperalgesia was induced by intradermal injection of epinephrine into the hindpaw of a mouse or rat. Epinephrine-induced hyperalgesia is a model system for the study of naturally occurring hyperalgesia, and the clinical relevance o this system is supported by the fact that local administration of epinephrine exacerbates symptoms in patients with neuropathic pain (B. Cboi, M. C. Rowbotham, *Pain* **69**, 55-63 (1997)) and that epinephrine causes anginal pain in the absence of apparent ischemia (B. Eriksson et al., *Am. J Cardiol.* 75, 241-245 (1995)). Epinephrine causes dose-dependent mechanical and thermal hyperalgesia in sensory nerve terminals in the skin by binding to β-adrenergic receptors on these nerve terminals. The β-adrenorgic receptors bound by epinephrine in turn activate two independent second messenger pathways, the PKC pathway and the cyclic AMP ("cAMP")/protein kinase A ("PKA") pathway. Although epinephrine-induced hyperalgesia is not mediated by prostaglandins, both epinephrine and prostaglandin E₂ ("PGE₂"), enhance the tetrodotoxin-resistant sodium current (TTX-RI_{Na}), which is important in inflammatory mediator-induced hyperalgesia and nociceptor sensitization. Since PKC inhibitors reduce the effect of epinephrine on that current, TTX-R I_{Na} can be a target of PKC, as well as ofPKA.

The direct action of epinephrine on primary sensory afferent neurons is in contrast to other hygeralgesia-inducing agents which indirectly sensitize these neurons. For instance, bradykinin and norepinephrine affect nociceptors by causing intermediary cells to release prostaglandins that act on nociceptors and by causing sympathetic neurons to send signals to nociceptors (N. Y. Andreev, N. Dimitrieva, M. Koltzenburg, S. B. McMahon, *Pain* **63**, 109-115 (1995); S. H. Ferreira, et al., *Brit. J. Pharmacol* **121**, 883-888 (1997); Y. O. Taiwo, P. H. Heller, J. D. Levine, *Neuroscience* **39**, 523-S31 (1990)). The fact that neither inhibition of prostaglandin synthesis nor hindpaw sympathectomy (elimination of sympathetic innervation of the hindpaw) had any effect on the ability of epinephrine to induce hyperalgesia shows that epinephrine's sole mode of action is direct. Epinephrine's direct effects on the primary afferent nociceptor provide further evidence that it is a good model to explore the contribution of the PKC isozyme(s) involved in nociceptive signaling.

Another aspect of the invention is a method of measuring the ability of a compound to modulate pain, by selecting, as a test compound, a compound that modulates the activity of PKCε, and administering said test compound to a subject to determine whether pain is modulated as set out in the claims. Preferably, the compound will inhibit the activity of PKCε. The subject will be an animal commonly used in pain research and/or development. The ability of a test compound to inhibit, the activity of PKCε may be determined with suitable assays measuring PKCε's function. For example, responses such as its activity, e.g., enzymatic activity, or PKCε's ability to bind its ligand, adapter molecule or substrate may be determined in *in vitro* assays. Cellular assays can be developed to monitor a modulation of second messenger production, changes in cellular metabolism, or effects on enzymatic activity. These assays may be performed using conventional techniques developed for these purposes. Finally, the ability of a test compound to inhibit the function of PKCε will be measured in suitable animal models *in vivo.*

Preferred embodiments of the present invention include a composition as set out in the claims combining an inhibitor of PKCε with one or more additional pain-reducing agents.

An individual pain medication often provides only partially effective pain alleviation because it interferes with just one pain-transducing pathway out of many. For instance, experiments presented herein show that PKCε and PKA are both secondary messengers of epinephrine-induced hyperalgesia. The pain associated with this type of hyperalgesia can be more effectively addressed by inhibiting both PKCε and PKA than by inhibiting PKCε alone. Alternatively, PKCε inhibitors can be administered in combination with a pain-reducing (analgesic) agent that acts at a different point in the pain perception process. A PKCε inhibitor can minimize pain by altering the responses of nociceptors to noxious stimuli. One class of analgesics, such as NSAIDs, down-regulates the chemical messengers of the stimuli that are detected by the nociceptors and another class of drugs, such as opioids, alters the processing of nociceptive information in the CNS. Other analgesics are local anesthetics, anticonvulsants, and antidepressants. Administering one or more classes of drug in addition to PKCε inhibitors can provide more effective amelioration of pain. NSAIDs are preferred components of the composition of the invention. Preferred NSAIDs are aspirin, acetaminophen, ibuprofen, and indomethacin.

The term "lessening pain" as used herein comprises a process by which the level of pain a subject perceives is reduced relative to the level of pain the subject would have perceived were it not for the intervention. Where the subject is a person, the level of pain the person perceives can be assessed by asking him or her to describe the pain or compare it to other painful experiences. Alternatively, pain levels can be calibrated by measuring the subject's physical responses to the pain, such as the release of stress-related factors or the activity of pain-transducing nerves in the peripheral nervous system or the CNS. One can also calibrate pain levels by measuring the amount of a well characterized analgesic required for a person to report that no pain is present or for a subject to stop exhibiting symptoms of pain. Lessening pain can result from increasing the threshold at which a subject experiences a given stimulus as painful. It can result from inhibiting hyperalgesia, the heightened sensitivity to a noxious stimulus, and such inhibition can occur without impairing nociception, the subject's normal sensitivity to a noxious stimulus.

"A subject in need thereof" comprises an animal or person, preferably a person, expected to experience pain in the near future. Such animal or person may have a ongoing condition that is causing pain currently and is likely to continue to cause pain, or the animal or person has been, is or will be enduring a procedure or event that usually has painful consequences. Chronic painful conditions such as diabetic neuropathic hyperalgesia and collagen vascular diseases are examples of the first type; dental work, particularly in an area of inflammation or nerve damage, and toxin exposure (including exposure to chemotherapeutic agents) are examples of the latter type.

"An effective amount" comprises an amount that results in the lessening of pain. Such effective amount will vary from subject to subject depending on the subject's normal sensitivity to pain, its height, weight, age, and health, the source of the pain, the mode of administering the inhibitor of PKCε, the particular inhibitor administered, and other factors. As a result, it is advisable to empirically determine an effective amount for a particular subject under a particular set of circumstances.

"An inhibitor of the e isozyme of protein kinase C (PKCε)" comprises a molecule or group of molecules that interferes with: (1) the expression, modification, regulation or activation of PKCε, (2) one or more of the normal functions of PKCε, or (3) the expression, modification, regulation or activation of a molecule acting downstream of PKCε in a PKCε-dependent pathway. The normal functions of PKCε, many of which are activation-dependent, include the phosphorylation of substrates (i.e., the catalytic activity of PKCε), autophosphorylation, movement from one intracellular location to another upon activation (i.e., intracellular translocation), and binding to or release from one or more proteins that anchor PKCε in a given location. An inhibitor of PKCε can also inhibit other isozymes of PKC. However, a selective inhibitor of PKCε significantly inhibits one or more normal functions of PKCε at a concentration at which the other isozymes of PKC are not significantly inhibited. An inhibitor "acts directly on PKCε" when the inhibitor binds to PKCε via electrostatic or chemical interactions. Such interactions may or may not be mediated by other molecules. An inhibitor acts "indirectly on PKCε" when its most immediate effect is on a molecule, other than PKCε, that influences the expression, activation or functioning of PKCε or the downstream effects of PKCε.

A compound or molecule "modulates the activity of PKCε" if it affects (1) one or more of the normal functions of PKCε, or (2) the expression, modification, regulation, activation or degradation of PKCε or a molecule acting upstream of PKCε in a regulatory or enzymatic pathway. The normal functions of PKCε, many of which are activation-dependent, include the phosphorylation of substrates (i.e., the catalytic activity of PKCε), autophosphorylation, movement from one intracellular location to another upon activation (i.e., intracellular translocation), and binding to or release from one or more proteins that anchor PKCε in a given location.

The difference between "acute" and "chronic" pain is one of timing: acute pain is experienced soon (preferably within about 48 hours, more preferably within about 24 hours, most preferably within about 12 hours) after the occurrence of the event (such as inflammation or nerve injury) that led to such pain. By contrast, there is a significant time lag between the experience of chronic pain and the occurrence of the event that led to such pain. Such time lag is at least about 48 hours after such event, preferably at least about 96 hours after such event, more preferably at least about one week after such event.

"Neuropathic pain" comprises pain arising from conditions or events that result in nerve damage. "Neuropathy" comprises a disease process resulting in damage to nerves. "Causalgia" denotes a state of chronic pain following nerve injury or a condition or event, such are cardiac infarction, that causes referred pain. "Allodynia" comprises a condition in which a person experiences pain in response to a normally nonpainful stimulus, such as a gentle touch. An "analgesic agent" comprises a molecule or combination of molecules that causes a reduction in pain. An analgesic agent employs a mechanism of action other than inhibition of PKCε when its mechanism of action does not involve direct (via electrostatic or chemical interactions) binding to and reduction in the function of PKCε or any intracellular molecule in the PKCε pathway.

The specific items mentioned in the foregoing definitions represent preferred embodiments of the present invention.

### EXAMPLES

### Example 1. Epinephrine-Induced Hyperalgesia is Prostaglandin-Independent and is Mediated by β-Adrenergic Receptors, PKC, Protein Kinase A and µ Opioid Receptors

As shown in Figure 1A, intradermal injections of 1ng to 1µg epinephrine into the dorsal surface of the hindpaw of a rat produced a dose-dependent decrease (F=90.7, p<0.01) in the threshold at which the rat withdrew the paw in response to the application of a linearly increasing mechanical force by the Ugo Basile Analgesimeter (Stoelting, Chicago, IL). The onset latency of this epinephrine-induced hyperalgesia was brief - it was significant 2 minutes after 1µg epinephrine injection, reached a peak effect by 5 minutes (Figure 2A) and lasted approximately 2 hours (Figure 2B).

Since epinephrine has affinity for both α- and β- adrenergic receptors ("α-AR" and "β-AR", respectively), the contribution of α-AR to epinephrine-induced hyperalgesia was tested using phentolamine, an α-AR antagonist. Earlier studies showed that a similar dose of phentolamine had no effect on basal paw threshold in the normal rat but significantly reversed formalin-induced hyperalgesia and inhibited the ability of rolipram, an inhibitor of type IV phosphodiesterase, to prolong PGE₂-induced hyperalgesia (J. D. Levine et al., *Nature* **323**, 158-160 (1986); A. K. Ouseph et al., *Neurosci.* **64**, 769-776 (1995)). However, phentolamine did not significantly affect epinephrine-induced hyperalgesia (Figure 1A), demonstrating that α-AR does not mediate the hyperalgesic effects of epinephrine.

To examine the role of β-AR in epinephrine-induced hyperalgesia, propranolol, a β-AR antagonist, and isoproterenol, a selective β-AR agonist, were administered by intradermal injection. Isoproterenol produced dose-dependent hyperalgesia in the absence of epinephrine (Figure 3). Propranolol significantly attenuated both the hyperalgesia caused by isoproterenol and that due to epinephrine (Figures 3 and 1A, respectively). Thus, epinephrine produces hyperalgesia by activating β-AR.

Bradykinin and norepinephrine are known to cause hyperalgesia indirectly, by triggering other cells to produce prostaglandins that have a sensitizing effect on primary afferent neurons and by stimulating sympathetic neurons that influence nociceptors (J. D. Levine et al., *Nature* **323**, 158-160 (1986); Y. O. Taiwo, P. H. Heller, J. D. Levine, *Neurosci*. **39**, 523-531 (1990)). To test whether epinephrine also causes hyperalgesia indirectly, prostaglandin synthesis was inhibited by continuous treatment with 4mg/kg of indomethacin (n=8) and the contribution of sympathetic neurons was eliminated by sympathectomy (n=8). Neither indomethacin treatment nor sympathectomy had any effect on the ability of epinephrine to induce hyperalgesia (Figure 1B). This suggests that epinephrine acts directly on nociceptive DRG neurons.

The roles of PKC, protein kinase A ("PKA") and the µ-opioid receptor in mediating epinephrine-induced hyperalgesia were assessed by measuring the effects of: Rp-adenosine 3', 5'-cyclic monophosphate ("Rp-cAMPs") and WIPTIDE, both inhibitors of PKA; SQ22536, an inhibitor of adenylyl cyclase, an enzyme activated by PKA; bisindolymaleimide ("BIM"), a nonselective inhibitor of PKC isozymes; and [D-Ala²,N-Me-Phe⁴,Gly⁵-ol]-enkephalin ("DAMGO"), p-opioid receptor agonist. The dose for each inhibitor was 1µg, except for WIPTIDE, which was administered in a 100ng dose. DAMGO was co-injected with epinephrine whereas the other inhibitors were applied 15 minutes prior to epinephrine injection. As shown in Table 1, all of these inhibitors significantly attenuated epinephrine-induced hyperalgesia, showing that epinephrine-mediated hyperalgesia is mediated by the PKC and PKA pathways and by µ-opioid receptors. The same dose of BIM had no effect on PGE₂-induced hyperalgesia but significantly attenuated isoproterenol-induced hyperalgesia. In contrast, the same dose of WIPTIDE also significantly attenuated PGE₂-induced hyperalgesia and almost completely abolished isoproterenol-induced hyperalgesia. This shows a role for PKA in both prostaglandin- and β-AR-mediated hyperalgesia and a role for PKC in the latter only.

**TABLE 1: Percentage decrease in nociceptive threshold in response to epinephrine, PGE₂ or isoproterenol (each 100 ng) and effects of DAMGO, SQ22536, BIM, Rp-cAMPs (all 1 µg) or WIPTIDE (0.1 µg). A large numeric value means more hyperalgesia.**

| | **Epinephrine** | **PGE₂^{§}** | **Isoproterenol** |
|---|---|---|---|
| **Alone** | 29.6±2.1 | 33.6±1.9 | 25.8±0.9 |
| | (n=24) | (n=20) | (n=16) |
| DAMGO | 11.5 ± 3.1* | 11.1 ± 4.0** | |
| | (n=6) | (n=8) | |
| **SQ22536** | 9.5 ±2.4* | 10.0±2.9** | |
| | (n=8) | (n=11) | |
| **BIM** | 4.6 ± 2.1* | 33.4 ± 4.1 | 7.1 ± 2.4*† |
| | (n=6) | (n=6) | (n=12) |
| **Rp-cAMPs** | 13.9 ± 4.6* | 3.1 ± 5.2** | |
| | (n=6) | (n=6) | |
| **WIPTIDE** | 10.9 ± 3.6* | 9.4 ± 3.1** | 1 ± 2.8*† |
| | (n=6) | (n=6) | (n=6) |

| | | | |
|---|---|---|---|
| *Significantly different from epinephrine alone (P<0.01). *†Significantly different from isoproterenol alone (P<0.01). **Significantly different from PGE₂ alone (P<0.01). § PGE₂ data are from (S. G. Khasar et al., *Neurosci.* **64**, 1161-1165 (1995)) or (Y. O. Taiwo and J. D. Levine, *Neurosci*. **44**, 131-135(1991)), except for BIM and WIPTIDE. | | | |

### Example 2. Epinephrine Causes Epinephrine-Induced Hyperalgesia by Acting Directly on Primary Afferent Nociceptors

To test whether the epinephrine-induced hyperalgesia measured using behavioral models is due to the direct action of epinephrine on primary afferent nociceptors, whole-cell patch-clamp experiments were performed on dissociated, small diameter DRG neurons within 12-24 hours of plating. These neurons have been previously shown to serve as a model for peripheral nociceptor terminals (P. I. Baccaglini and P. G. Hogan, *Proc. Natl. Acad. Sci. USA* 80, 594-598 (1983); S. Pitchford and J. D. Levine, *Neurosci. Let.* 132, 105-108 (1991); S. England et al., *J. Physiol. London* **495***,* 429-440 (1996); M. S. Gold et al., *Neurosci.* **71**, 265-275 (1996)).

Current-clamp recordings were performed using the perforated-patch whole-cell technique. The number of action potentials generated during a 750ms ramp-and-plateau depolarizing current injection, as well as the latency to the first spike, were used as a measure of excitability. After 5-10 minutes of baseline recordings, epinephrine (1 µM) was added to the bath. Figure 4A shows voltage traces from a typical neuron before and during exposure to 1µM epinephrine, while Figure 4B shows the time course of changes in the number of action potentials and the latency to the first action potential for another cell. For 11 neurons treated with 1µM epinephrine, the average number of action potentials generated in response to the current ramp-and-plateau was 1.7±0.2 before the addition of epinephrine and 5.3±0.9 five minutes or more after the start of drug perfusion (p<0.005). When no epinephrine was present, the mean latency from the start of current injection to the peak of the first spike was 278±42ms; following epinephrine perfusion, the mean latency became 189±21ms (n=11, p<0.05). Nine (81%) of the 11 neurons tested showed an increase in spike number and 5 (45%) of these neurons also showed a decrease in spike latency of at least 50ms. Mean resting potentials were unaffected by epinephrine.

The fact that the addition of 10µM of propranolol 30 seconds prior to and with 1µM epinephrine abolished the increase in spike number (1.2±0.1 before and 1.3±0.2 after epinephrine addition, n=7, p<0.05) and decrease in first-spike latency (235±9ms before and 233±10ms after epinephrine addition, n=7, p<0.05) usually caused by epinephrine demonstrates that, like in the behavioral studies, epinephrine acts on primary afferent neurons by activating β-AR.

Since hyperalgesic agents that sensitize nociceptors *in vitro* have been shown to increase TTX-RI_{Na} (S. England et al., *J*. *Physiol. London* **495**, 429-440 (1996); M. S. Gold et al., *Proc. Natl. Acad. Sci. USA* **93**, 1108-1112 (1996)), epinephrine's effect on TTX-RI_{Na} was examined. As shown in Figure 5, 1µM epinephrine caused a marked potentiation of TTX-RI_{Na}, shifting the current-voltage plots for activation by approximately 10mV in the hyperpolarized direction (Figure 5B). This dose of epinephrine caused an increase in TIX-RI_{Na} in 14 out of 21 (67%) neurons. Since isoproterenol application produced similar results as epinephrine (Figure 5C) and 2µM propranolol eliminated this potentiation but 5µM phentolamine did not, epinephrine's effects on TTX-RI_{Na} are mediated by β-AR and not α-AR.

The roles of PKC and PKA in mediating epinephrine-induced potentiation of TTX-RI_{Na} were tested by the addition of 100µM Rp-cAMPs or 100µM BIM. Rp-cAMPs abolished and BIM significantly decreased (n=11, p<0.01) epinephrine-induced potentiation TTX-RI_{Na} (Figures 7 and 8), thus demonstrating involvement of the PKA and PKC pathways. PKC inhibition by BIM decreased the number of neurons (5 out of 11 v. 8 out of 11) responsive to epinephrine and the magnitude of their responses (mean peak current increase of 32% v. 49%).

Thus, the epinephrine-induced hyperalgesia displayed by animals is due to epinephrine's stimulation of β-adrenergic receptors and PKC and PKA pathways in primary afferent nociceptors.

### Example 3. Hyperalgesic Responses to Mechanical and Thermal Stimuli as well as Acetic Acid Injection are Significantly Diminished in the Absence of PKCε

One of the most powerful methods for testing the role of a protein in a process is to generate mutant animals that lack the protein and to compare the process in mutant animals with that in their wild-type counterparts. To examine whether PKCε plays a role in pain signaling, homozygous PKCε-mutant mice were generated by homologous recombination, production of chimeric mice and subsequent breeding. The construction of these mice is described in detail in the U.S. Provisional Patent Application entitled "PKCε as Modulator of Anxiety" and filed on July 6, 1998. PKCε-mutant mice are viable and cannot be distinguished from wild-type littermates in the normal cage environment. Moreover, hematoxylin and eosin staining of the brain, spinal cord and DRG neurons showed no anatomical abnormalities.

Mechanical, thermal and chemical nociception were evaluated in wild-type and PKCε-mutant mice. For both wild-type (n=8) and PKCε-mutant (n=8) mice, the basal mechanical nociceptive threshold was measured as the frequency at which mice withdrew their paws after being poked in the hindpaw with a von Frey Hair ("VFH"; Stoelting Co.) applying a force of 4, 6.9 or 14.8 grams. As shown in Figure 9A, the percent-withdrawal response after VFH stimulation was similar in wild-type and mutant mice. When 100ng of epinephrine or prostaglandin E₂ ("PGE₂") was administered by intraplantar injection prior to VFH stimulation, the percent-withdrawal response to a particular intensity of VFH stimulus dramatically increased in wild-type mice, indicating that the injected compounds lower nociceptive thresholds (E. Kinnman, J. D. Levine, *Neuroscience* 64, 751-767 (1995)). While mutant mice and wild-type mice demonstrated similar percent-withdrawal responses to VFH stimulation after exposure to PGE₂, the percent-withdrawal response following epinephrine injection was significantly lower (p=0.0013 for 4 gram VHF; p=0.0692 for 6.9 gram VHF; and p=0.0023 for 14.8gram VHF) in PKCε-mutant mice than in wild-type mice (Figure 9A).

When thermal nociceptive thresholds were determined by the Hargreave thermal nociceptive test (K. O. Aley, D. B. Reichling, J. D. Levine, *Neuroscience* **73***,* 259-265 (1996)), wild-type (n=8) and mutant (n=8) mice demonstrated similar basal thresholds and similar responses to PGE₂, but PKCε-mutant animals displayed significantly reduced (p=0.0006) epinephrine-induced thermal hyperalgesia compared to their wild-type littermates (Figure 9B).

Chemically induced hyperalgesia in wild-type (n=4) and mutant (n=4) mice was determined by the acetic acid writhing test (S. J. Ward, A. E. Takemori, *J*. *Pharmacol. Exp. Ther.* **224**, 525-530 (1983)). As demonstrated in Figure 9C, PKCε-mutant mice have significantly higher (p=0.0124) nociceptive thresholds than wild-type animals.

The presence of normal PGE₂-mediated responses in PKCε-mutant mice is consistent with data indicating that PGE₂ hyperalgesia and sensitization is not dependent on PKC, but instead is dependent on PKA (G. R. Lewin. A. M. Ritter. L. M. Mendell. *J*. *Neurosci.* **13**, 2136-2148 (1993)). These studies suggest that a defect in PKCε-mediated signal transduction accounts for reduced epinephrine-mediated hyperalgesia in PKCε-mutant mice. The lower score in the acetic acid writhing test in PKCε-mutant mice also suggests that PKCε contributes to inflammatory pain. The findings that PKCε does not contribute to baseline nociceptive thresholds and that the mutant mice act and appear normal indicate that inhibitors of PKCε can ameliorate pain without causing serious systemic side effects or interfering with normal nociceptive responses.

### Example 4. Administration of a Selective Inhibitor of PKCε Lessens Hyperalgesia in Response to a Mechanical Stimulus

To confirm that the hyperalgesic responses of PKCε-mutant mice reported in Example 3 are due to PKCε's role in these processes rather than a possible requirement for PKCε during development of the nervous system, the nociceptive and hyperalgesic responses of wild-type adult (200-250gm) Sprague-Dawley rats obtained from Bantin-Kingman (Fremont CA) were examined immediately following treatment with a selective inhibitor of PKCε, the εV1-2 peptide. This peptide selectively inhibits the ε isozyme of PKC by interfering with its activation-induced translocation- in the presence of εV1-2, the binding of PKCε to β'COP, its anchoring protein, is impaired (J. A. Johnson, M. O. Gray, C.-H. Chen, D. Mochly-Rosen, *J. Biol*. *Chem.* **271,** 24962-24966 (1996); M. Csukai, C.-H. Cehn, M. A. De Matteis, D. Mochly-Rosen, *J. Biol. Chem.* **272**, 29200-29206 (1997); B. Hundle, et al., *J. Biol*. *Chem.* **272,** 15028-15035 (1997)).

For mechanical nociceptive testing, the nociceptive flexion reflex was quantified using an Ugo Basile analgesymeter that applies a constantly increasing force measured in grams (Stoelting, Chicago, IL; K. O. Aley, J. D. Levine, J. *Neurosci.* **17**, 8018-23 (1997)). The mean basal mechanical threshold for rats used in these experiments was 105.3+/-2.8 grams. The effects of hyperalgesia-inducing agents epinephrine (100ng) and PGE₂ (100ng) on mechanical nociceptive thresholds were determined by measuring paw withdrawal frequencies at 15, 20, and 25 minutes after administration of the hyperalgesic agent and then calculating the mean of these three readings. As shown in Figure 10E, the mechanical nociceptive thresholds of rats (n=8) that received footpad injections of sterile water (to create a hypotonic environment) and 1µg of a scrambled version of the εV1-2 peptide prior to epinephrine treatment were very similar to those of rats (n=8) treated only with epinephrine; both groups showed an approximately 25-30% threshold reduction. In contrast, rats (n=8) that received footpad injections of sterile water (to create a hypotonic environment) and 1µg of the εV1-2 peptide prior to epinephrine treatment, exhibited significantly less epinephrine-induced threshold reduction (Figure 10E). As expected, rats that received εV1-2 and PGE₂ (n=10) showed threshold reductions that were indistinguishable from those of rats receiving just PGE₂ (n=10). These results demonstrate that inhibition of PKCε in adult neurons reduces epinephrine-mediated hyperalgesia.

### Example 5. PKCε Is The Only PKC Isozyme Involved In Epinephrine-Induced Hyperalgesia

To assess the contribution of PKC isozymes other than PKCε to epinephrine-induced hyperalgesia, we co-injected epinephrine with a PKC inhibitor that is not isozyme-selective, bisindolymaleimide I ("BIM", D. Toullec, et *al*., *J*. *Biol*. *Chem.* **266**, 15771-15781 (1991)), and compared the resulting nociceptive thresholds with those recorded following successive injections of the εV1-2 peptide and epinephrine. Because BIM inhibits epinephrine-induced hyperalgesia to a similar extent as εV1-2 (Figure 10D), PKCε appears to be the only PKC isozyme involved in epinephrine-induced hyperalgesia.

### Example 6. PKCε Acts in Primary Afferent Neurons

In order to determine the location(s) of PKCε's hyperalgesia-transducing activity, the cellular distribution of PKCε was examined. Anesthetized mice and rats were transcardially perfused with PBS (137 mM NaCl, 2.7 mM KCl, 1.47 mM KH₂PO₄, 8 mM Na₂HPO₄, 0.5 mM MgCl₂, 0.9 mM CaCl₂, pH 7.2) and then 4% paraformaldehyde in PBS. Spinal cords and DRGs were removed and placed in 4% paraformaldehyde for 3 hours, then in PBS-containing 20% sucrose for 24 hr and 40% sucrose for 24 hr. Sections (5-10 µm) were cut using a refrigerated microtome (Leica SM2000R), and treated with methanol:acetone (1:1) for 10 minutes at -20°C. DRG neurons were incubated at 25°C in PBS/1% normal goat serum/0.05% Tween-20 for 1 hour, and then in the same buffer containing polyclonal anti PKCε (1:300 dilution, Santa Cruz Biotechnology) for 1-2 hours. Sections were visualized using FITC-conjugated goat anti-rabbit IgG (1:50 dilution, Vector Laboratories, Burlingame, CA). For PKCε immunocytochemistry, spinal cord sections were pretreated with 3% H₂O₂ before incubation with 4% normal goat serum and 0.05% Tween-20 in Superblock (Pierce). Specimens were then incubated with polyclonal anti-PKCε antibody (1:300 dilution) overnight in PBS/1% Tween-20, and immunoreactivity was detected using the ABC kit (Vector) with diaminobenzamine followed by enhancement with 0.02% osmium tetroxide.

PKCε was present in 70 ± 2% of the DRG neurons in wild-type adult mice (n=6), including most small- and many medium-diameter DRG neurons (Figure 11A and C). In rats, similar size DRG neurons express PKCε (Figure 10A).

Since nociceptive DRG neurons terminate in the dorsal horn of the spinal cord, PKCε distribution was also examined in the spinal cords of wild-type and PKCε-mutant mice. In normal mouse (n=4) spinal cords, PKCε was detected in the superficial dorsal horn (Figure 3D). Thus, PKCε is present in primary afferent nerve fibers of DRG neurons. In PKCε -mutant mice, no PKCε was detected in the spinal cord (Figure 11B).

To address the possibility that intradermal injection of the εV1-2 peptide or BIM blocked hyperalgesia by inhibiting PKCε in non-neuronal cells residing in the dermis, the skin of normal rats was stained with a PKCε antibody. PKCε was detected in a thin band in the basal layer of the epidermis, but no PKCε was detectable in the dermis. Preabsorption of the anti-PKCε antibody with the immunizing peptide eliminated the staining, demonstrating that it is specific. These results indicate that, since no PKCε is present in the dermis, intradermally injected PKCε inhibitors do not act by inhibiting PKCε in dermal connective tissue. Rather, these data combined with those presented above lead to the conclusion that PKCε inhibitors injected into the dermis enter primary afferent neurons (small- and medium-diameter DRG neurons) and act on the PKCε present in these cells.

PKCε immunoreactivity was present in small and medium sized DRG neurons and in the superficial laminae of the dorsal horn of the spinal cord where nociceptive afferents terminate. Moreover, intradermal injection of a PKCε inhibitor peptide reduced epinephrine-induced hyperalgesia; this peptide was injected after the injection of hypotonic solution, an effective method for introducing peptides into peripheral nerve endings (L. K. West, L. Huang, *Biochemistry* **19**, 4418-4423 (1980)). Since other cell types in the dermis did not express PKCε, these results indicate that PKCε residing in DRG neurons is necessary for the hyperalgesic effect of epinephrine.

### Example 7. PKCε Modulates Acute and Chronic Chemotherapy-Induced Neuropathic Pain

Humans undergoing chemotherapy frequently experience hyperalgesia. Vincristine is a chemotherapeutic drug known to cause such hyperalgesia, and a rat model of vincristine-induced hyperalgesia was established as follows. Vincristine (100 µg/kg in saline; Sigma Chemical Catalog) was administered intravenously daily to each of 32 rats over a period of two weeks. After the second day of vincristine administration, the rats demonstrated an acute dose-dependent decrease in mechanical nociceptive thresholds and an increased response ("hyperalgesia") to non-noxious mechanical stimuli, administered by Ugo Basile analgesymeter, relative to control rats but did not show any significant motor deficits (as measured by Rotorod analysis). During the second week of vincristine administration, vincristine-treated rats displayed lowered chronic mechanical nociceptive thresholds and increased response to non-noxious mechanical stimuli 24 hours after the most recent vincristine treatment. Responses to mechanical stimuli gradually decreased to baseline during the two weeks following discontinuation of vincristine treatment.

To determine whether administration of an inhibitor of PKCε would lessen vincristine-induced hyperalgesia in the rat model described above, on the 13th day of vincristine treatment, rats received no treatment (n= 16) or intradermal injections of sterile water followed by intradermal injections of either 1µg εV1-2, a PKCε inhibitory peptide (n=8; labeled "PKCεI" on Figure 12A), or 1µg S-VεV1-2, a scrambled version of the εV1-2 peptide (n=8; labeled "PCKεI SCR" on Figure 12A), 20-30 minutes before administration of a mechanical stimulus. As shown in Figure 12A, vincristine-induced chronic hyperalgesia was significantly diminished by administration of the PKCε inhibitor (p<0.05) but unaffected by the scrambled inhibitor peptide. These data demonstrate that inhibition of PKCε lessens pain due to toxic nerve injury, such as that induced by a chemotherapeutic drug.

### Example 8. PKCε Modulates Pain Associated with Diabetic Neuropathy

Persons with uncontrolled or poorly controlled diabetes frequently experience hyperalgesia. The role of PKCε in diabetic neuropathy was examined in a model of diabetes in which male Sprague-Dawley rats receive subcutaneous injection of streptozotocin (70 mg/kg), a pancreatic β-cell toxin. These rats display hyperglycemia and glucosuria within 24 hours after the injection and show decreased nociceptive thresholds in response to mechanical stimuli administered by Ugo Basile analgesymeter in the hind paw after one week (Ahlgren, S.C. et al, *J. Neurophysiology,* **68**, 2077-2085 (1992); Ahlgren, S.C. and Levine, J.D., *Neuroscience* **52**, 1049-1055 (1993)). Four weeks after the streptozotocin injection, each rat received no treatment (n=8) or an intradermal injection of sterile water followed by a intradermal injections of (1) 1 µg bisindolymaleimide, a protein kinase C inhibitor (n=4; labeled "BIM" on Figure 12B), (2) 1 µg εV1-2, a PKCε inhibitory peptide (n=8; labeled "PKCεI" on Figure 12B), or (3) 1 µg S-VεV1-2, a scrambled version of the εV1-2 peptide (n=4; labeled "PCKε1 SCR" on Figure 12B) and were subsequently exposed to mechanical stimuli. As shown in Figure 12B, administration of either bisindolymaleimide or εV1-2 markedly reduced the diabetes-induced decrease in nociceptive threshold (both p<0.05) but treatment with the scrambled peptide had little effect on the decrease in nociceptive threshold. These data demonstrate that PKCε is the major¹
¹ Since bisindolymaleimide caused somewhat greater reduction in the percentage decrease in nociceptive threshold than PKCεI, it is possible that one or more isozymes of PKC other than PKCε have roles in modulating diabetes-induced hyperalgesia. isozyme of PKC that mediates hyperalgesia in diabetics and that administration of inhibitors of PKCε lessens pain associated with diabetic neuropathy.

### Example 9. PKCε Modulates Hyperalgesia Induced by Traumatic Nerve Injury

The Seltzer traumatic nerve injury model (Seltzer, Z. et al, *Pain* **43**, 205-218 (1990)) is used to investigate the role of PKCs in modulating hyperalgesia induced by traumatic nerve injury. After determining sites in the dorsal aspect of the hindpaw in which traumatic nerve injury has caused hyperalgesia, rats receive no treatment or intradermal injections of sterile water at such site followed by intradermal injections of (1) 1 µg bisindolymaleimide, (2) 1µg εV1-2, or (3) 1µg S-VεV1-2. Responses to mechanical stimuli are subsequently tested, and inhibitors of PKCε are shown to at least partially reverse hyperalgesia associated with traumatic nerve injury.

### Example 10. PKCε Modulates Acute Inflammatory Pain

The acetic acid writhing experiments reported in Example 3 indicate that PKCε contributes to inflammatory pain. To investigate whether PKCε mediates hyperalgesia induced by other inflammatory agents, rats received intradermal 10µl injections of a 1% solution of carrageenan, a potent irritant (Ferreira, S.H. et al, *Nature* 334, 698-700 (1988)) and three and a half hours later, the rats received no treatment (n=12; labeled "Car-4" on Figure 13) or intradermal injections of sterile water followed by intradermal injections of either 1 µg εV1-2, a PKCε inhibitory peptide (n=6; labeled "εV" on Figure 13), or 1µg S-VεV1-2, a scrambled version of the εV1-2 peptide (n=6; labeled S-εV" on Figure 13). When mechanical stimuli were applied 30 minutes later, as shown in Figure 13, the rats that received only carrageenan or both carrageenan and the scrambled peptide showed substantial hyperalgesia. By comparison, the rats that received carrageenan and the PKCε inhibitory peptide showed almost no mechanical hyperalgesia. These data demonstrate that administration of an inhibitor of PKCε significantly diminishes carrageenan-induced hyperalgesia.

### Example 11. PKCε Modulates Chronic Inflammatory Pain

### A. Model for Chronic Inflammatory Pain

In order to investigate the role, if any, of PKCε in chronic inflammatory pain, the following model system for chronic inflammatory pain was developed. Rats received intradermal hind paw injections of carrageenan, an inflammatory agent. For up to 3-4 days following carrageenan administration, the rats displayed mechanical hyperalgesia, as assessed by the Randall-Selitto paw-withdrawal test. This was considered an acute response to the carrageenan-induced inflammation. Although administration of Prostaglandin E₂ ("PGE₂"), 5-hydroxytryptamine ("5-HT," serotonin) or CGS-21680 (an adenosine A₂-selective receptor agonist) normally causes untreated rats (that is, rats that have not received carrageenan injections) to experience hyperalgesia lasting up to 6 hours, the hyperalgesia experienced by carrageenan-sensitized rats following administration of any one of these substances lasted up to 24 hours. This prolonged hyperalgesia was seen in such rats up to three weeks after the carrageenan injection. These data establish a model of chronic prolonged inflammatory pain and suggest that changes induced by acute inflammation persist for a long period of time.

### B. PKCε Modulates Chronic Inflammatory Pain and Processes That Give Rise To It

The role of protein kinase C enzymes generally and the PKCε isozyme in particular in modulating chronic inflammatory pain was tested by administering 1µg bisindolymaleimide ("BIM," a nonselective PKCε inhibitor) or 1µg εV1-2 (a peptide that selectively inhibits PKCε) several days after the carrageenan injection, and subsequently treating the rats with PGE₂ and measuring the resulting hyperalgesia. The prolonged PGE₂-induced hyperalgesia normally seen in carrageenan-sensitized rats was inhibited by administration of BIM or εV1-2. These data show that prolonged PGE₂-induced hyperalgesia is dependent upon PKCε activity and demonstrate that administration of an inhibitor of PKCε lessens chronic enhanced pain due to prior inflammation.

To examine whether protein kinase C modulates the processes by which a brief inflammatory event causes prolonged hyperalgesia many days later, rats received carrageenan and 1µg BIM injections at the same time. Hyperalgesia was measured 72 hours later, when the rats were treated PGE₂. Administration of BIM at the same time as carrageenan attenuated both the acute hyperalgesia normally induced by carrageenan exposure and the prolonged hyperalgesia normally observed when the rats are treated with PGE₂ 72 or more hours after carrageenan exposure. These data demonstrate that PKC modulates both acute and chronic hyperalgesia. Therefore, administration of an inhibitor of PKC either at approximately the same time as the inflammatory agent giving rise to chronic, prolonged hyperalgesia or at about the same time as the hyperalgesia-inducing stimulus can lessen such chronic hyperalgesia. Administration of εV1-2 in lieu of BIM confirmed that these effects are due to the PKCε isozyme and showed that administration of a selective inhibitor of PKCε immediately before, during or after an inflammatory event lessens both acute pain due to such an event and interferes with the mechanisms that lead to chronic pain following such an event.

## Claims

1. An inhibitor of the ε isozyme of protein kinase C (PKCε) for use in a method of lessening pain, said method comprising:
administering an effective amount of an inhibitor of the ε isozyme of protein kinase C (PKCε), wherein said effective amount is one that does not significantly inhibit other isozymes of PKC, and
wherein said inhibitor causes said lessening of pain by interfering with the activity of PKCε, and
wherein said inhibitor is a peptide selected from the group consisting of: εV1-1, εV1-2, εV1-3, εV1-4, εV1-5 and εV1-6.

2. The inhibitor of claim 1, wherein said administration results in decreased hyperalgesia without reduced nociception.

3. The inhibitor of claim 1, wherein said method also comprises administering a compound from the group consisting of: an inhibitor of protein kinase A (PKA), an inhibitor of CAMP, a nonsteroidal anti-inflammatory drug, a local anesthetic, an anticonvulsant, an antidepressant, and an opioid.

4. The inhibitor of claim 1, wherein said pain is acute or chronic.

5. The inhibitor of claim 1, wherein said pain is inflammatory or neuropathic.

6. The inhibitor of claim 5, wherein
said inflammatory pain is due to sunburn, osteoarthritis, colitis, carditis, dermatitis, myositis, neuritis or collagen vascular disease, and
said neuropathic pain is due to causalgia, diabetes, collagen vascular disease, trigeminal neuralgia, spinal cord injury, brain stem injury, thalamic pain syndrome, cancer, chronic alcoholism, stroke, abscess, demyelinating disease, herpes infection, AIDS, trauma, surgery, amputation, toxin or chemotherapy.

7. A method of measuring the ability of a test compound to modulate pain, said method comprising:
selecting, as a test compound, an inhibitor of the ε isozyme of protein kinase C (PKCε), wherein the inhibitor significantly inhibits PKCε at a concentration at which the other isozymes of PKC are not significantly inhibited, and
measuring the ability of the inhibitor to modulate pain in an animal model of pain,
wherein said test compound modulates pain in said subject by modulating the activity of PKCε.

8. A composition comprising an inhibitor of the ε isozyme of protein kinase C (PKCε), wherein the inhibitor significantly inhibits PKCε at a concentration at which the other isozymes of PKC are not significantly inhibited, and an analgesic agent, said analgesic agent having a mechanism of action other than inhibition of PKCε, wherein said inhibitor is a peptide selected from the group consisting of: εV1-1, εV1-2, εV1-3, εV1-4, εV1-5 and εV1-6.

9. The composition of claim 8, wherein said analgesic agent is selected from the group consisting of: a nonsteroidal anti-inflammatory drug, an opioid, a local anesthetic, an anticonvulsant and an antidepressant.

10. The use of an inhibitor of the ε isozyme of protein kinase C (PKCε), wherein the inhibitor significantly inhibits PKCε at a concentration at which the other isozymes of PKC are not significantly inhibited, in the preparation of a medicament for the treatment of pain, and wherein said inhibitor causes said treatment of pain by interfering with the activity of PKCε.

11. The use of claim 10, wherein said inhibitor is a selective inhibitor of PKCε.

12. The use of claim 11, wherein said selective inhibitor is a peptide selected from the group consisting of: εV1-1, εV1-2, εV1-3, εV1-4, εV1-5 and εV1-6.

13. The use of claim 10, wherein said inhibitor results in decreased hyperalgesia without reduced nociception.

14. The use of claim 10, wherein said medicament also comprises a compound from the group consisting of: an inhibitor of protein kinase A (PKA), an inhibitor of cAMP, a nonsteroidal anti-inflammatory drug, a local anesthetic, an anticonvulsant, an antidepressant, and an opioid.

15. The use of claim 10, wherein said pain is acute or chronic.

16. The use of claim 10, wherein said pain is inflammatory or neuropathic.

17. The use of claim 16, wherein
said inflammatory pain is due to sunburn, osteoarthritis, colitis, carditis, dermatitis, myositis, neuritis or collagen vascular disease, and
said neuropathic pain is due to causalgia, diabetes, collagen vascular disease, trigeminal neuralgia, spinal cord injury, brain stem injury, thalamic pain syndrome, cancer, chronic alcoholism, stroke, abscess, demyelinating disease, herpes infection, AIDS, trauma, surgery, amputation, toxin or chemotherapy.

## Patentansprüche

1. Inhibitor des ε-Isozyms von Protein-Kinase C (PKCε) zur Verwendung in einem Verfahren zur Linderung von Schmerz, wobei das Verfahren Folgendes umfasst:
das Verabreichen einer wirksamen Menge eines Inhibitors des ε-Isozyms von Protein-Kinase C (PKCε), worin die wirksame Menge eine Menge ist, die keine anderen Isozyme von PKC signifikant hemmt, und
worin der Inhibitor Schmerz lindert, indem er die Aktivität von PKCε beeinflusst, und
worin der Inhibitor ein Peptid ist, das aus der aus εV1-1, εV1-2, εV1-3, εV1-4, εV1-5 und εV1-6 bestehenden Gruppe ausgewählt ist.

2. Inhibitor nach Anspruch 1, worin die Verabreichung zu verringerter Hyperalgesie ohne verringerte Nozizeption führt.

3. Inhibitor nach Anspruch 1, worin das Verfahren auch die Verabreichung einer Verbindung umfasst, die aus der aus einem Inhibitor von Protein-Kinase A (PKA), einem Inhibitor von cAMP, einem entzündungshemmenden Nichtsteroid-Arzneimittel, einem Lokalanästhetikum, einem Antikonvulsivum, einem Antidepressivum und einem Opioid bestehenden Gruppe ausgewählt ist.

4. Inhibitor nach Anspruch 1, worin der Schmerz akut oder chronisch ist.

5. Inhibitor nach Anspruch 1, worin der Schmerz entzündlich oder neuropathisch ist.

6. Inhibitor nach Anspruch 5, worin
der entzündliche Schmerz durch einen Sonnenbrand, Osteoarthritis, Colitis, Carditis, Dermatitis, Myositis, Neuritis oder Kollagenose ausgelöst ist,
der neuropatische Schmerz durch Kausalgie, Diabetes, Kollagenose, Trigeminusneuralgie, eine Rückenmarksschädigung, eine Hirnstammschädigung, das Thalamussyndrom, Krebs, chronischen Alkoholismus, einen Schlaganfall, einen Abszess, eine Entmarkungskrankheit, eine Herpesinfektion, AIDS, ein Trauma, eine Operation, eine Amputation, ein Toxin oder Chemotherapie ausgelöst ist.

7. Verfahren zur Messung der Fähigkeit einer Testverbindung, Schmerz zu lindern, wobei das Verfahren Folgendes umfasst:
das Auswählen eines Inhibitors des ε-Isozyms von Protein-Kinase C (PKCε) als Testverbindung, worin der Inhibitor PKCε in einer Konzentration signifikant hemmt, bei der die anderen Isozyme von PKC nicht signifikant gehemmt werden, und
das Messen der Fähigkeit des Inhibitors, Schmerz in einem Schmerz-Tiermodell zu lindern,
worin die Testverbindung Schmerz in einem Individuum lindert, indem die Aktivität von PKCε moduliert wird.

8. Zusammensetzung, die einen Inhibitor des ε-Isozyms von Protein-Kinase C (PKCε), worin der Inhibitor PKCε in einer Konzentration signifikant hemmt, bei der die anderen Isozyme von PKC nicht signifikant gehemmt werden, und ein Analgetikum umfasst, wobei das Analgetikum einen anderen Wirkmechanismus aufweist als die Hemmung von PKCε, worin der Inhibitor ein aus der aus εV1-1, εV1-2, εV1-3, εV1-4, εV1-5 und εV1-6 bestehenden Gruppe ausgewähltes Peptid ist.

9. Zusammensetzung nach Anspruch 8, worin das Analgetikum aus der aus einem entzündungshemmenden Nichtsteroid-Arzneimittel, einem Opioid, einem Lokalanästhetikum, einem Antikonvulsivum und einem Antidepressivum ausgewählt ist.

10. Verwendung eines Inhibitors des ε-Isozyms von Protein-Kinase C (PKCε), worin der Inhibitor PKCε in einer Konzentration signifikant hemmt, bei der die anderen Isozyme von PKC nicht signifikant gehemmt werden, bei der Herstellung eines Medikaments zur Schmerzbehandlung, worin der Inhibitor den Schmerz behandelt, indem er die Aktivität von PKCε beeinflusst.

11. Verwendung nach Anspruch 10, worin der Inhibitor ein selektiver Inhibitor von PKCε ist.

12. Verwendung nach Anspruch 11, worin der selektive Inhibitor ein aus der aus εV1-1, εV1-2, εV1-3, εV1-4, εV1-5 und εV1-6 bestehenden Gruppe ausgewähltes Peptid ist.

13. Verwendung nach Anspruch 10, worin der Inhibitor zu verringerter Hyperalgesie ohne verringerte Nozizeption führt.

14. Verwendung nach Anspruch 10, worin das Medikament auch eine Verbindung umfasst, die aus der aus einem Inhibitor von Protein-Kinase A (PKA), einem Inhibitor von cAMP, einem entzündungshemmenden Nichtsteroid-Arzneimittel, einem Lokalanästhetikum, einem Antikonvulsivum, einem Antidepressivum und einem Opioid bestehenden Gruppe ausgewählt ist.

15. Verwendung nach Anspruch 10, worin der Schmerz akut oder chronisch ist.

16. Verwendung nach Anspruch 10, worin der Schmerz entzündlich oder neuropathisch ist.

17. Verwendung nach Anspruch 16, worin
der entzündliche Schmerz durch einen Sonnenbrand, Osteoarthritis, Colitis, Carditis, Dermatitis, Myositis, Neuritis oder Kollagenose ausgelöst ist,
der neuropatische Schmerz durch Kausalgie, Diabetes, Kollagenose, Trigeminusneuralgie, eine Rückenmarksschädigung, eine Hirnstammschädigung, das Thalamussyndrom, Krebs, chronischen Alkoholismus, einen Schlaganfall, einen Abszess, eine Entmarkungskrankheit, eine Herpesinfektion, AIDS, ein Trauma, eine Operation, eine Amputation, ein Toxin oder Chemotherapie ausgelöst ist.

## Revendications

1. Inhibiteur de l'isozyme ε de la protéine kinase C (PKCε) à utiliser dans un procédé pour soulager la douleur, ledit procédé comprenant les étapes consistant à:
administrer une quantité efficace d'un inhibiteur de l'isozyme ε de la protéine kinase C (PKCε), ladite quantité efficace étant une quantité qui n'inhibe pas significativement d'autres isozymes de la PKC, et
dans lequel ledit inhibiteur provoque ledit soulagement de la douleur en interférant sur l'activité de la PKCε, et
dans lequel ledit inhibiteur est un peptide choisi dans le groupe consistant en: εV1-1, εV1- 2, εV1-3, εV1-4, εV1-5 et εV1-6.

2. Inhibiteur selon la revendication 1, dans lequel ladite administration entraîne une diminution de l'hyperalgésie sans diminution de la nociception.

3. Inhibiteur selon la revendication 1, dans lequel ledit procédé comprend aussi l'administration d'un composé provenant du groupe consistant en: un inhibiteur de la protéine kinase A (PKA), un inhibiteur du cAMP, un médicament anti-inflammatoire non stéroidien, un anesthésique local, un anti-convulsivant, un anti-dépresseur et un opioïde.

4. Inhibiteur selon la revendication 1, dans lequel ladite douleur est aiguë ou chronique.

5. Inhibiteur selon la revendication 1, dans lequel ladite douleur est inflammatoire ou neuropathique.

6. Inhibiteur selon la revendication 5, dans lequel
ladite douleur inflammatoire est due à un coup de soleil, à l'arthrose, à la colite, à la cardite, à la dermatite, à la myosite, à la névrite ou à une maladie du collagène vasculaire, et
ladite douleur neuropathique est due à une causalgie, au diabète, à une maladie du collagène vasculaire, à la névralgie du trijumeau, à une lésion de la moelle épinière, à une lésion du tronc cérébral, à un syndrome de douleur thalamique, à un cancer, à l'alcoolisme chronique, à un AVC, à un abcès, à une maladie de démyélinisation, à une infection herpétique, au SIDA, à un traumatisme, à une opération chirurgicale, à une amputation, à une toxine ou à une chimiothérapie.

7. Procédé pour mesurer la capacité d'un composé à l'essai à moduler la douleur, ledit procédé comprenant les étapes consistant à:
sélectionner, comme composé à l'essai, un inhibiteur de l'isozyme s de la protéine kinase C (PKCε), l'inhibiteur inhibant significativement la PKCε à une concentration à laquelle les autres isozymes de la PKC ne sont pas significativement inhibées, et à
mesurer la capacité de l'inhibiteur à moduler la douleur dans un modèle animal de douleur,
le composé à l'essai modulant la douleur chez ledit sujet en modulant l'activité de la PKCε.

8. Composition comprenant un inhibiteur de l'isozyme ε de la protéine kinase C (PKCε), l'inhibiteur inhibant significativement la PKCε à une concentration à laquelle les autres isozymes de la PKC ne sont pas significativement inhibées, et un agent analgésique, ledit agent analgésique ayant un mécanisme d'action autre que l'inhibition de la PKCε, ledit inhibiteur étant un peptide sélectionné dans le groupe consistant en: εV1-1, εV1-2, εV1-3, εV1-4, εV1-5 et εV1-6.

9. Composition selon la revendication 8, dans laquelle ledit agent analgésique est choisi dans le groupe consistant en: un médicament anti-inflammatoire non stéroïdien, un opioïde, un anesthésique local, un anti-convulsivant et un anti-dépresseur.

10. Utilisation d'un inhibiteur de l'isoenzyme ε de la protéine kinase C (PKCε), l'inhibiteur inhibant significativement la PKCε à une concentration à laquelle les autres isozymes de la PKC ne sont pas significativement inhibées, dans la préparation d'un médicament pour le traitement de la douleur, et ledit inhibiteur entraînant ledit traitement de la douleur en interférant sur l'activité de la PKC ε.

11. Utilisation selon la revendication 10, dans laquelle ledit inhibiteur est un inhibiteur sélectif de la PKCε.

12. Utilisation selon la revendication 11, dans laquelle ledit inhibiteur sélectif est un peptide choisi dans le groupe consistant en: εV1-1, εV1-2, εV1-3, εV1-4, εV1-5 et εV1-6.

13. Utilisation selon la revendication 10, dans laquelle ledit inhibiteur entraîne une diminution de l'hyperalgésie sans diminution de la nociception.

14. Utilisation selon la revendication 10, dans laquelle ledit médicament comprend aussi un composé du groupe consistant en: un inhibiteur de la protéine kinase A (PKA), un inhibiteur du cAMP, un médicament anti-inflammatoire non stéroïdien, un anesthésique local, un anti-convulsivant, un anti-dépresseur, et un opioïde.

15. Utilisation selon la revendication 10, dans laquelle ladite douleur est aiguë ou chronique.

16. Utilisation selon la revendication 10, dans laquelle ladite douleur est inflammatoire ou neuropathique.

17. Utilisation selon la revendication 16, dans laquelle
ladite douleur inflammatoire est due à un coup de soleil, à l'arthrose, à la colite, à la cardite, à la dermatite, à la myosite, à la névrite ou à une maladie du collagène vasculaire, et
ladite douleur neuropathique est due à une causalgie, au diabète, à une maladie du collagène vasculaire, à la névralgie du trijumeau, à une lésion de la moelle épinière, à une lésion du tronc cérébral, à un syndrome de douleur thalamique, à un cancer, à l'alcoolisme chronique, à un AVC, à un abcès, à une maladie de démyélinisation, à une infection herpétique, au SIDA, à un traumatisme, à une opération chirurgicale, à une amputation, à une toxine ou à une chimiothérapie.
